# EUROPÄISCHE PATENTANMELDUNG

(11) **EP 2 932 858 A1**
(43) Veröffentlichungstag der Anmeldung: **21.10.2015**
(21) Anmeldenummer: 14165020.0
(22) Anmeldetag: 16.04.2014
(51) Int. Cl.: A23L 1/22, A23L 1/226, C07C 69/732

(54) **Homovanillinsäure-Ester, insbesondere zum Erzielen eines Wärme- und/oder Schärfeeindrucks**

(71) Anmelder: Symrise AG, 37603 Holzminden (DE)
(72) Erfinder: Backes, Dr. Michael, 37603 Holzminden (DE); Ley, Dr. Jakob Peter, 37603 Holzminden (DE); Degenhardt, Dr. Andreas, Maidenhead, Berkshire SL64LL (GB); Paetz, Susanne, 37671 Höxter (DE); Reichelt, Dr. Katharina, 37603 Holzminden (DE); Riess, Thomas, 37603 Holzminden (DE); Klose, Bettina, 38124 Braunschweig (DE); Hentschel, Dr. Fabia, 37603 Holzminden (DE)
(74) Vertreter: Eisenführ Speiser

(57) **Zusammenfassung**

Die vorliegende Erfindung betrifft insbesondere neue Verwendungen von Verbindungen der Formel (I) (wie hierin beschrieben), zum Teil auch neue Verbindungen der Formel (I) als solche, Verbindungen der Formel (I) enthaltende Aromakompositionen, neue Zubereitungen sowie neue Verfahren unter Verwendung von Verbindungen der Formel (I). Weitere Aspekte der vorliegenden Erfindung ergeben sich aus den Patentansprüchen und der nachfolgenden Beschreibung samt Beispielen.

## Beschreibung

Die vorliegende Erfindung betrifft insbesondere neue Verwendungen von Verbindungen der Formel (I) (wie hierin beschrieben), zum Teil auch neue Verbindungen der Formel (I) als solche, Verbindungen der Formel (I) enthaltende Aromakompositionen, neue Zubereitungen sowie neue Verfahren unter Verwendung von Verbindungen der Formel (I). Weitere Aspekte der vorliegenden Erfindung ergeben sich aus den Patentansprüchen und der nachfolgenden Beschreibung samt Beispielen.

Capsaicin [N-(4-Hydroxy-3-methoxybenzyl)-8-methyl-(6E)-nonensäureamid] und andere Capsaicinoide wie Nonivamid ([N-(4-Hydroxy-3-methoxybenzyl)-nonansäureamid) sind als scharf schmeckende und wärmeerzeugende Aromastoffe aus verschiedenen Capsicum-Arten, insbesondere Chilipfeffer, schon seit langem bekannt. Bei entsprechend geringer Dosierung der Capsaicinoide wird eine neutrale Schärfe und ein Wärmegefühl im Mund wahrgenommen, wobei die Schwelle zum schmerzhaften Schärfe- und Hitzeempfinden sehr schnell überschritten wird. Der Einsatz von Capsaicin in Lebensmitteln ist allerdings in der Europäischen Union nicht erlaubt (wurde von der Community Flavoring List in 2004 gestrichen), da das genotoxische Potential der Verbindung negativ bewertet wurde (European Food Safety Authority (EFSA), P., Italy, Opinion of the Scientific Committee on Food on Capsaicin. European Comission 2002, (SDF/CS/FLAV/FLAVOUR/8 ADD1 Final)). Zudem ist der Einsatz in Lebensmitteln oft schwierig, da Capsaicin einen sehr niedrigen Geschmacksschwellenwert und eine hohe Potenz als Scharfstoff (16,000,000 Scoville units, vgl. http://en.wikipedia.org/wiki/Capsaicin; Version des Eintrags mit letzten Änderungen vom 11. November 2011, 21:02 Uhr) hat. Capsaicin wird, auch auf Grund des hohen Preises des Reinstoffs, zudem nahezu ausschließlich in Form eines Capsicumextrakts verwendet, der neben weiteren Scharfstoffen noch Reste anderer, nach Capsicum schmeckender oder riechender Aromastoffe enthält und daher für den breiten Einsatz nur bedingt geeignet ist. Somit besteht trotz der guten sensorischen Eigenschaften ein Bedarf an weniger problematischen Scharfstoffen.

Das im weißen Pfeffer vorkommende Piperin (1-Piperoylpiperidin) verursacht zwar auch einen starken scharfen Eindruck (Römpp Lexikon Naturstoffchemie, Thieme 1997, S. 500), zeigt aber im Vergleich zu Capsaicin eine relative Scharfe von nur ca. 1 %. Darüber hinaus besitzt Piperin einen intensiven Eigengeschmack, der an Pfeffer erinnert, so dass die Anwendung in vielen Zubereitungen nur beschränkt erfolgen kann.

Auf Grund des lipophilen Charakters dieser vanilloiden Scharfstoffe ist der Einsatz des Schärfeeindrucks oft um einige Sekunden verzögert und hält auch besonders lange an, insbesondere in wenig lipophile Bestandteile (z.B. Triglyceride) enthaltenden Zubereitungen, wobei hier gleichzeitig die Löslichkeit nur unzureichend ist. Ähnliches gilt für Scharfstoffe wie Gingerol-[6] aus Ingwer oder Paradol-[6] aus Paradieskörnern, die beide zwar scharf schmecken, aber ein starken Nachgeschmack haben.

Zwar sind weitere (z.B. Starkenmann, C.; Cayeux, I.; Birkbeck, A. A., Exploring Natural Products for New Taste Sensations. Chimia 2011, 65, (6), 407-410), scharf schmeckende Stoffe wie das Driman Polygodial (aus Tasmanischem Pfeffer, *Tasmannia lanceolata*) oder Resiniferatoxin aus *Euphorbia resinifera* bekannt (Szallasi, A.; Biro, T.; Modarres, S.; Garlaschelli, L.; Petersen, M.; Klush, A.; Vidari, G.; Jonassohn, M.; De Rosa, S.; Sterner, O.; Blumberg, P. M.; Krause, J. E., Dialdehyde sesquiterpenes and other terpenoids as vanilloid. Eur. J. Pharmacol. 1998, 356, 81-89); die Drimane sind aber auf Grund ihrer Dialdehyd-Struktur nur begrenzt einsatzfähig, da sie mit freien Aminogruppen z.B. von Proteinen reagieren und somit ihre Wirkung einbüßen und das Resiniferatoxin ist stark toxisch und nicht geeignet für die menschliche Ernährung. Zudem sind diese Stoffe ebenfalls stark lipophil.

Der Methylester der Homovanillinsäure wurde in verschiedenen Hölzern, welche zur Lagerung von Wein und Spirituosen verwendet werden, nachgewiesen (z. B. Fernandez de Simon, B.; Esteruelas, E.; Munoz, A. M.; Cadahia, E.; Sanz, M., J. Agric. Food Chem. 2009, 57, 3217-3227.). Der Ethylester der Homovanillinsäure wurde hingegen in Wein und Spirituosen selber nachgewiesen, meistens in Zusammenhang mit einer Lagerung in Eichenfässern (z.B. Cabaroglu, T.; Canbas, A.; Baumes, R.; Bayonove, C.; Lepoutre, J. P.; Günata, Z., J. Food Sei. 1997, 62, 680-692. van Jaarsveld, F. P.; Hattingh, S.; Minnaar, P., S. Afr. J. Enol. Vitic. 2009, 30, 24-37.). Die geringen Konzentrationen von z.B. 2 µg/L reichen aber nicht, um einen wärme und/oder schärfeerzeugenden Effekt hervorzurufen.

In US 2009/0170942 A1 sind bestimmte Homovanillinsäure-Ester-Derivate und diverse (medizinische) Anwendungen davon beschrieben.

Bei einer Untersuchung der Schmerzempfindung verschiedenster Capsaicin-Derivate in Rattenaugen wurden Methyl-, Propyl-, Octyl-, Nonyl- und Dodecylester der Homovanillinsäure getestet und als aktiv bewertet (Szolcsanyi, J.; Jancso-Gabor, A., Arzneim.-Forsch.(Drug. Res.) 1975, 25, 1877-1881).

Im Gegensatz zu den oben genannten Scharfstoffen ist Ethanol ein kleines hydrophiles Molekül, das einen schnellen und angenehmen Schärfeeindruck verursacht, der aber nicht sehr lange vorhält. Da dies erst bei relativ hohen Konzentrationen von 0.5 % und mehr funktioniert, der Genuss von Ethanol aber gesundheitlich problematisch ist und bei dauerhaften Aufnahme auch zur Sucht führen kann, wird nach Aromaformulierungen gesucht, die den Schärfeverlauf von Ethanol simulieren können, ohne dabei die genannten Nachteile zu zeigen. Für diese Anwendung sind schon einige Scharfstoffe beschrieben worden, so z.B. wurden in EP 1,515,943-B1 bestimmte längerkettige Vanillomandelsäurealkylamide oder in WO 2009 065,239 Polygodial und Warburganal als Scharfstoffe zur Erzielung eines Ethanol-ähnlichen Schärfegefühls beschrieben; durch die ebenfalls vorhandene Lipophilie ist wiederum ein langanhaltender Schärfeeindruck zu beobachten, der von den Testern nicht als Ethanol-typischen Eindruck beschrieben wird.

Somit besteht der Bedarf an weniger lipophilen, sensorisch schnell einsetzenden und nicht langanhaltenden Scharfstoffen, die einen wärmenden Geschmackseindruck hervorrufen. Besonderer Bedarf besteht an Stoffen, die oben genannte Eigenschaften besitzen und natürlich vorkommen oder in lebensmittelüblichen Prozessen aus natürlich vorkommenden Lebensmittelbestandteilen oder Aromastoffen gebildet werden.

Diesem Bedarf kann nun erfindungsgemäß durch Verwendung einer Verbindung der Formel (I) 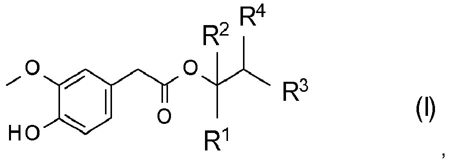 wobei
(i) R¹ und R² unabhängig voneinander ein Wasserstoffatom oder einen Alkylrest mit 1-2 Kohlenstoffatomen darstellen,
   R³ und R⁴ unabhängig voneinander ein Wasserstoffatom oder einen linearen oder verzweigten Alkylrest mit 1 bis 5 Kohlenstoffatomen (beispielsweise ausgewählt aus der Gruppe bestehend aus Methyl, Ethyl, 1-Propyl, 2-Propyl-, 1-Butyl, 2-Butyl, tert-Butyl, 2-Methylprop-1-yl, 1-, 2- oder 3-Pentyl-, 2-Methylbut-1-yl, 2-Methylbut-2yl, 3-Methylbut-1-yl und 3-Methylbut-2-yl, vorzugsweise aus Methyl, Ethyl, 1-Propyl, 2-Propyl, 1-Butyl, 2-Butyl, tert-Butyl, 2-Methylprop-1-yl und 1-Pentyl), einen Phenylrest, einen Alkylphenylrest oder einen linearen oder verzweigten Alkenylrest mit 2 bis 4 Kohlenstoffatomen (beispielsweise ausgewählt aus der Gruppe bestehend aus Ethenyl, Prop-2en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, und die jeweiligen gegebenenfalls möglichen Z- und E-Isomere) oder einen Alkenylphenylrest darstellen,
   oder
(ii) R¹ und R³ zusammen mit den sie verknüpfenden Kohlenstoffatomen einen Cyclohexylring bilden (die Reste R² und R⁴ sind dann Substituenten des Cycloalkylrings; s. hierzu z.B. Formel (la) weiter unten)), der optional mit einem zusätzlichen Rest R⁵ substituiert ist, wobei R⁵ ein Alkylrest mit 1-2 Kohlenstoffatomen ist,
   R² ein Wasserstoffatom oder einen Alkylrest mit 1-2 Kohlenstoffatomen darstellt,
   R⁴ ein Wasserstoffatom oder einen linearen oder verzweigten Alkylrest mit 1 bis 5 Kohlenstoffatomen (beispielsweise ausgewählt aus der Gruppe bestehend aus Methyl, Ethyl, 1-Propyl, 2-Propyl-, 1-Butyl, 2-Butyl, tert-Butyl, 2-Methylprop-1-yl, 1-, 2- oder 3-Pentyl-, 2-Methylbut-1-yl, 2-Methylbut-2yl, 3-Methylbut-1-yl und 3-Methylbut-2-yl, vorzugswweise aus Methyl, Ethyl, 1-Propyl, 2-Propyl, 1-Butyl, 2-Butyl, tert-Butyl, 2-Methylprop-1-yl und 1-Pentyl), einen Phenylrest, einen Alkylphenylrest oder einen linearen oder verzweigten Alkenylrest mit 2 bis 4 Kohlenstoffatomen (beispielsweise ausgewählt aus der Gruppe bestehend aus Ethenyl, Prop-2en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, und die jeweiligen gegebenenfalls möglichen Z- und E-Isomere) oder einen Alkenylphenylrest darstellt,
oder eines physiologisch akzeptablen Salzes davon, wobei die phenolische Hydroxygruppe in Formel (I) deprotoniert ist, oder einer Mischung umfassend eine oder mehrere Verbindungen der Formel (I) und/oder physiologisch akzeptable Salze (insbesondere seine Natrium-, Kalium-, Ammonium-, Calcium-, Magnesium- oder Zinksalze) davon, wobei jeweils die phenolische Hydroxygruppe in Formel (I) deprotoniert ist, oder bestehend aus mehreren unterschiedlichen Verbindungen der Formel (I) und/oder Salzen davon, wobei jeweils die phenolische Hydroxygruppe in Formel (I) deprotoniert ist,
als Aromastoff Rechnung getragen werden.

Im Falle einer Mischung von unterschiedlichen Verbindungen der Formel (I) (wie hierin beschrieben) gilt im Rahmen des vorliegenden Textes, dass die unterschiedlichen Verbindungen z.B. nicht nur Verbindungen mit unterschiedlicher Summenformel, sondern auch verschiedene Stereoisomere mit identischer Summenformel sein können.

Die hierin beschriebenen Verbindungen der Formel (I) eignen sich insbesondere
- als Aromastoff bzw. Scharfstoff mit einem wärme- und/oder schärfeerzeugenden Effekt, d.h. als Stoff, der sensorisch einen Wärmeeindruck hervorrufen kann,
   und/oder
- als Aromastoff zum Vermindern oder Maskieren eines unangenehmen Geschmackseindrucks, vorzugsweise ausgewählt aus der Gruppe bestehend aus adstringierend, bitter, trocken, staubig, mehlig, kalkig und metallisch (weitere Details hierzu ergeben sich aus den Ausführungen weiter unten),
   und/oder
- als Aromastoff zum Verstärken eines angenehmen Geschmackseindrucks, vorzugsweise ausgewählt aus der Gruppe bestehend aus wärmend, scharf und kühlend (weitere Details hierzu ergeben sich aus den Ausführungen weiter unten).

An dieser Stelle ist anzumerken, dass die hierin beschriebenen Vorteile und Effekte der Verbindungen der Formel (I) in der Regel für deren Salze (wie hierin beschrieben) entsprechend gelten.

Bevorzugt ist eine Verwendung wie oben beschrieben, wobei für die Verbindung der Formel (I) bzw. eine, mehrere oder sämtliche Verbindungen der Formel (I) unabhängig voneinander in der Mischung gilt:
(i) R¹ und R² stellen unabhängig voneinander ein Wasserstoffatom oder Methyl dar,
   R³ und R⁴ stellen unabhängig voneinander ein Wasserstoffatom oder einen linearen oder verzweigten Alkylrest mit 1 bis 5 Kohlenstoffatomen (beispielsweise wie oben beschrieben) dar,
   oder
(ii) Formel (I) entspricht der folgenden Formel (la) 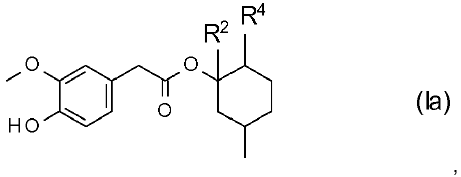 R² stellt ein Wasserstoffatom dar,
   R⁴ stellt 2-Propyl dar.

Bevorzugt ist auch eine Verwendung wie oben beschrieben, wobei für die Verbindung der Formel (I) bzw. eine, mehrere oder sämtliche Verbindungen der Formel (I) unabhängig voneinander in der Mischung gilt:
R¹ und R² stellen jeweils ein Wasserstoffatom dar,
R³ stellt ein Wasserstoffatom oder einen linearen oder verzweigten Alkylrest mit 1 bis 4 Kohlenstoffatomen dar,
R⁴ stellt ein Wasserstoffatom dar.

Besonders bevorzugt ist die Verbindung der Formel (I) bzw. sind eine, mehrere oder sämtliche Verbindungen der Formel (I) in der Mischung ausgewählt bzw. aus der Gruppe bestehend aus
2-Phenylethyl-2-(4-hydroxy-3-methoxy-phenyl)acetat (**1**) 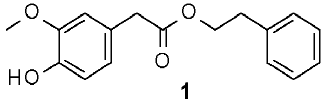
[(*E*)-Cinnamyl]-2-(4-hydroxy-3-methoxy-phenyl)acetat (**2**) 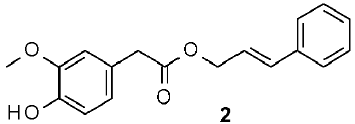
1-Ethylbutyl-2-(4-hydroxy-3-methoxy-phenyl)acetat (**3**) 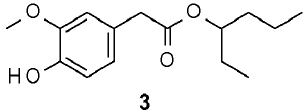
3-Methylbut-2-enyl-2-(4-hydroxy-3-methoxy-phenyl)acetat (**4**) 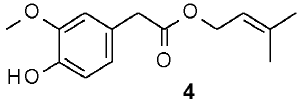
[(*E*)-Hex-2-enyl]-2-(4-hydroxy-3-methoxy-phenyl)acetat (**5**) 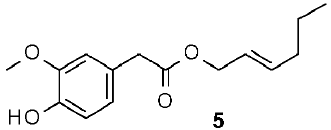
[(Z)-Hex-3-enyl]-2-(4-hydroxy-3-methoxy-phenyl)acetat (**6**) 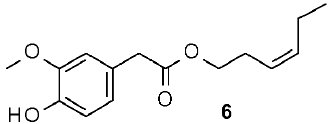
Isopropyl-2-(4-hydroxy-3-methoxy-phenyl)acetat (**7**) 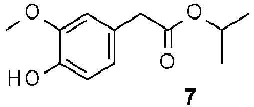
*sec*-Butyl-2-(4-Hydroxy-3-methoxy-phenyl)acetat (**8**) 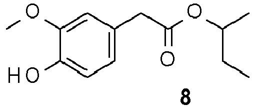
Isobutyl-2-(4-hydroxy-3-methoxy-phenyl)acetat (**9**) 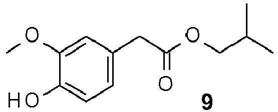
1,1-Dimethylpropyl 2-(4-hydroxy-3-methoxy-phenyl)acetat (**10**) 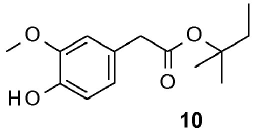
Isopentyl-2-(4-hydroxy-3-methoxy-phenyl)acetat (**11**) 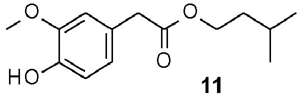
2-Methylbutyl-2-(4-hydroxy-3-methoxy-phenyl)acetat (**12**) 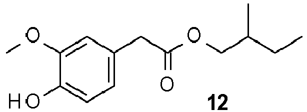
1-Methylpentyl-2-(4-hydroxy-3-methoxy-phenyl)acetat (**13**) 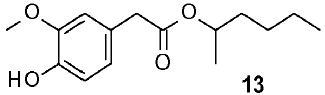
Heptyl-2-(4-hydroxy-3-methoxy-phenyl)acetat (**14**) 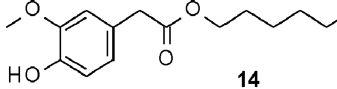
1-Methylhexyl-2-(4-hydroxy-3-methoxy-phenyl)acetat (**15**) 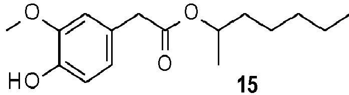
(2-Isopropyl-5-methyl-cyclohexyl)-2-(4-hydroxy-3-methoxy-phenyl)acetat (**16**) 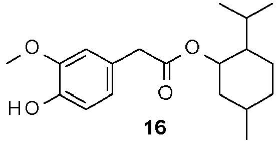
Ethyl-2-(4-hydroxy-3-methoxy-phenyl)acetat (**17**) 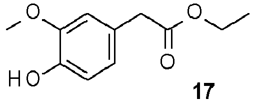
Propyl-2-(4-hydroxy-3-methoxy-phenyl)acetat (**18**) 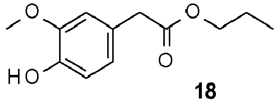
Butyl-2-(4-hydroxy-3-methoxy-phenyl)acetat (**19**) 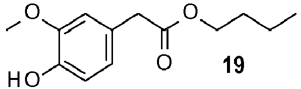
Pentyl-2-(4-hydroxy-3-methoxy-phenyl)acetat (**20**) 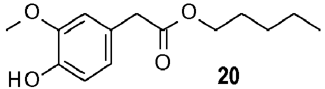
Hexyl-2-(4-hydroxy-3-methoxy-phenyl)acetat (**21**)

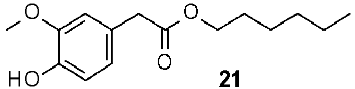

Die hierin beschriebenen Verbindungen eignen sich vorteilhafterweise zur Verwendung (insbesondere wie oben) in einer pharmazeutischen, einer der Ernährung, der Mundhygiene oder dem Genuss dienenden Zubereitung, vorzugsweise wobei die Gesamtmenge an Verbindung(en) der Formel (I) und/oder Salz(en) davon in der Zubereitung ausreicht, um
(a) bei Gebrauch oder Verzehr der Zubereitung sensorisch einen wärmenden und/oder scharfen Effekt auf der Zunge oder im Mundraum hervorzurufen,
   und/oder
(b) einen unangenehmen Geschmackseindruck, vorzugsweise ausgewählt aus der Gruppe bestehend aus adstringierend, bitter, trocken, staubig, mehlig, kalkig und metallisch, zu vermindern oder zu maskieren (weitere Details hierzu ergeben sich aus den Ausführungen weiter unten),
   und/oder
(c) einen angenehmen Geschmackseindruck, vorzugsweise ausgewählt aus der Gruppe bestehend aus wärmend, scharf und kühlend, zu verstärken (weitere Details hierzu ergeben sich aus den Ausführungen weiter unten).

Dabei ist es gemäß einem speziellen Aspekt der vorliegenden Erfindung bevorzugt, wenn die Gesamtmenge an Verbindung(en) der Formel (I) und/oder Salz(en) davon in der Zubereitung nicht ausreicht, um einen wärmenden oder scharfen Effekt auf der Zunge oder im Mundraum hervorzurufen, aber ausreicht, um einen unangenehmen Geschmackseindruck eines unangenehm schmeckenden Stoffes oder Stoffgemisches zu maskieren oder zu vermindern.

Ein weiterer Aspekt der vorliegenden Erfindung betrifft neue Verbindungen der Formel (I), Salze davon deren Mischungen, nämlich eine Verbindung der Formel (I) oder ein physiologisch akzeptables Salz davon, wobei die phenolische Hydroxygruppe in Formel (I) deprotoniert ist, oder eine Mischung umfassend eine oder mehrere unterschiedliche Verbindungen der Formel (I) und/oder ein oder mehrere physiologisch akzeptable Salze davon, wobei die phenolische Hydroxygruppe in Formel (I) jeweils deprotoniert ist, oder bestehend aus mehreren unterschiedlichen Verbindungen der Formel (I) und/oder physiologisch akzeptablen Salzen davon, wobei die phenolische Hydroxygruppe in Formel (I) jeweils deprotoniert ist, 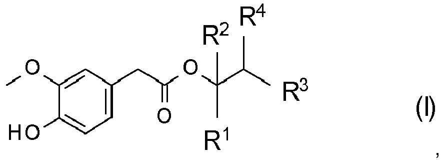 wobei
(i) R¹ und R² unabhängig voneinander ein Wasserstoffatom oder einen Alkylrest mit 1-2 Kohlenstoffatomen darstellen,
   R³ und R⁴ unabhängig voneinander ein Wasserstoffatom oder einen linearen oder verzweigten Alkylrest mit 1 bis 5 Kohlenstoffatomen (beispielsweise wie oben beschrieben), einen Phenylrest, einen Alkylphenylrest oder einen linearen oder verzweigten Alkenylrest mit 2 bis 4 Kohlenstoffatomen (beispielsweise wie oben beschrieben) oder einen Alkenylphenylrest darstellen,
   oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
   R² ein Wasserstoffatom oder einen Alkylrest mit 1-2 Kohlenstoffatomen darstellt,
   R⁴ ein Wasserstoffatom oder einen linearen oder verzweigten Alkylrest mit 1 bis 5 Kohlenstoffatomen (beispielsweise wie oben beschrieben), einen Phenylrest, einen Alkylphenylrest oder einen linearen oder verzweigten Alkenylrest mit 2 bis 4 Kohlenstoffatomen (beispielsweise wie oben beschrieben) oder einen Alkenylphenylrest darstellt,
mit der Maßgabe, dass
- R¹, R², R³ und R⁴ nicht sämtlich Wasserstoffatome darstellen,
und
- für den Fall, dass R¹, R² und R⁴ Wasserstoff darstellen, R³ weder einen linearen Alkylrest mit 1, 2, 4 oder 5 Kohlenstoffatomen (entsprechende Alkenylreste sind nicht ausgenommen), noch 2-Propyl, Phenyl oder Ethylphenyl darstellt,
und
- für den Fall, dass R², R³ und R⁴ Wasserstoff darstellen, R¹ nicht einen linearen Alkylrest mit 1 oder 2 Kohlenstoffatomen darstellt,
- nicht R³ und R⁴ Methyl darstellen, wenn R¹ und R² Wasserstoff darstellen, vorzugsweise weder R³, noch R⁴ Methyl darstellen, wenn R¹ und R² Wasserstoff darstellen,
und
- nicht R¹ und R² Methyl darstellen, wenn R³ und R⁴ Wasserstoff darstellen, vorzugsweise weder R1, noch R2 Methyl darstellen, wenn R³ und R⁴ Wasserstoff darstellen.

Besonders bevorzugt ist es dabei, wenn die Verbindung de Formel (I) bzw. eine, mehrere oder sämtliche Verbindungen der Formel (I) in der Mischung ausgewählt ist bzw. sind aus der Gruppe bestehend aus
[(*E*)-Cinnamyl]-2-(4-hydroxy-3-methoxy-phenyl)acetat (**2**) 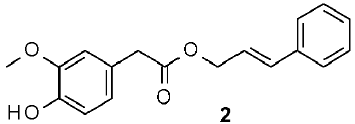
1-Ethylbutyl-2-(4-hydroxy-3-methoxy-phenyl)acetat (**3**) 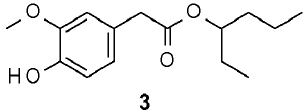
3-Methylbut-2-enyl-2-(4-hydroxy-3-methoxy-phenyl)acetat (**4**) 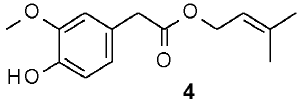
[(*E*)-Hex-2-enyl]-2-(4-hydroxy-3-methoxy-phenyl)acetat (**5**) 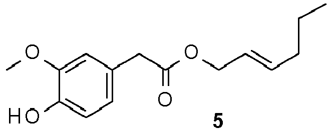
[(Z)-Hex-3-enyl]-2-(4-hydroxy-3-methoxy-phenyl)acetat (**6**) 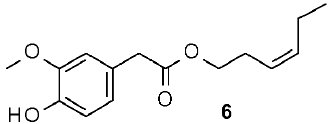
1,1-Dimethylpropyl 2-(4-hydroxy-3-methoxy-phenyl)acetat (**10**) 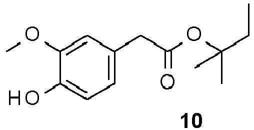
2-Methylbutyl-2-(4-hydroxy-3-methoxy-phenyl)acetat (**12**) 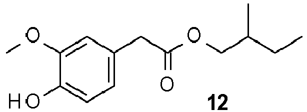
1-Methylpentyl-2-(4-hydroxy-3-methoxy-phenyl)acetat (**13**) 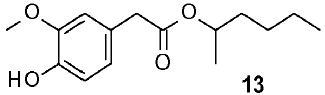
1-Methylhexyl-2-(4-hydroxy-3-methoxy-phenyl)acetat (**15**) 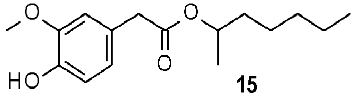
(2-Isopropyl-5-methyl-cyclohexyl)-2-(4-hydroxy-3-methoxy-phenyl)acetat (**16**) 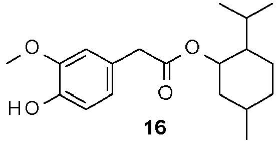
Pentyl-2-(4-hydroxy-3-methoxy-phenyl)acetat (**20**)

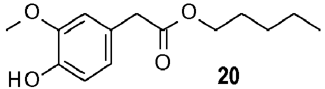

Im Übrigen gilt für die neuen Verbindungen der Formel (I) und deren Salze sowie Mischungen davon das oben im Zusammenhang mit den erfindungsgemäß zu verwendenden Verbindungen, Salzen und Mischungen Gesagte entsprechend.

Im Rahmen der vorliegenden Erfindung wurde festgestellt, dass Verbindungen der Formel (I) bzw. deren Salze und Mischungen davon, insbesondere ab Konzentrationen von 1.0 mg/kg, einen schnell aufkommenden und wenig lang anhaltenden, angenehmen Schärfe- bzw. Wärme- und leicht stechende Eindrücke vermitteln.

Die vorliegende Erfindung betrifft demnach insbesondere auch neue Aromakompositionen, nämlich eine Aromakomposition
(A) umfassend oder bestehend aus einer neuen, erfindungsgemäßen Mischung wie oben beschrieben,
   vorzugsweise wobei
   die Gesamtmenge an Verbindung(en) der Formel (I) und/oder Salz(en) davon in der Aromakomposition, bezogen auf das Gesamtgewicht der Aromakomposition, im Bereich von 500 bis 100.000 mg/kg liegt, vorzugsweise im Bereich von 1.000 bis 40.000 mg/kg, besonders bevorzugt im Bereich von 1.000 bis 15.000 mg/kg
   oder
(B) umfassend eine Verbindung der Formel (I), wie oben im Zusammenhang mit einer erfindungsgemäßen Verwendung definiert, oder ein physiologisch akzeptables Salz davon, wie oben im Zusammenhang mit einer erfindungsgemäßen Verwendung definiert, oder umfassend oder bestehend aus einer Mischung wie oben im Zusammenhang mit einer erfindungsgemäßen Verwendung definiert,
   wobei
   die Gesamtmenge an Verbindung(en) der Formel (I) und/oder Salz(en) davon in der Aromakomposition, bezogen auf das Gesamtgewicht der Aromakomposition, im Bereich von 500 bis 100.000 mg/kg liegt, vorzugsweise im Bereich von 1.000 bis 40.000 mg/kg, besonders bevorzugt im Bereich von 1.000 bis 15.000 mg/kg,
vorzugsweise zudem umfassend einen oder mehrere weitere, nicht der Formel (I) entsprechende Aromastoffe, beispielsweise ausgewählt aus der Gruppe bestehend aus
a) Wärme verursachende oder Scharfstoffe, bevorzugt ausgewählt aus der Liste bestehend aus: Capsaicinoiden, wie zum Beispiel Capsaicin, Dihydrocapsaicin oder Nonivamid; Gingerolen, wie zum Beispiel Gingerol-6, Gingerol-8, oder Gingerol-10; Shogaolen wie Shogaol-6, Shogaol-8, Shogaol-10; Gingerdionen, wie zum Beispiel Gingerdion-6, Gingerdion-8 oder Gingerdion-10; Paradolen, wie zum Beispiel Paradol-6, Paradol-8 oder Paradol-10; Dehydrogingerdionen, wie zum Beispiel Dehydrogingerdion-6, Dehydrogingerdion-8 oder Dehydrogingerdion-10; Piperin und Piperinderivaten;
b) als stechend oder beißend wahrnehmbare Stoffe, bevorzugt ausgewählt aus der Gruppe bestehend aus: aromatischen Isothiocyanaten, wie zum Beispiel Phenylethylisothiocyanat, Allylisothiocyanat, Cyclopropylisothiocyanat, Butylisothiocyanat, 3-Methylthiopropylisothiocyanat, 4-Hydroxybenzylisothiocyanat, 4-Methoxybenzylisothiocyanat;
c) als kribbelnd (tingling) beschriebenen Alkamide, vorzugsweise ausgewählt aus der Gruppe bestehend aus 2E,4E-Decadiensäure-N-isobutylamid (trans-Pellitorin) (insbesondere solche wie beschrieben in WO 2004/043906, die bezüglich der darin offenbarten entsprechenden Verbindungen auf dem Wege der Verweisung Bestandteil dieser Anmeldung wird); 2E,4Z- Decadiensäure-N-isobutylamid (cis-Pellitorin) (insbesondere solche wie beschrieben in WO 2004/000787, die bezüglich der darin offenbarten entsprechenden Verbindungen auf dem Wege der Verweisung Bestandteil dieser Anmeldung wird); 2Z,4Z- Decadiensäure-N-isobutylamid; 2Z,4E- Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy-2-methylpropyl)amid (gamma-Hydroxysanshool); 2E,4E,8Z,10E,12E-Tetradecapentaensäure-N-(2-hydroxy-2-methylpropyl)amid (gamma-Hydroxysanshool); 2E,4E,8E,10E,12E-Tetradecapentaensäure-N-(2-hydroxy-2-methylpropyl)amid (gamma-Hydroxyisosanshool); 2E,4E,8Z,10E,12E-Tetradecapentaensäure-N-(2-methyl-2-propenyl)amid (gamma-Dehydrosanshool); 2E,4E,8Z,10E,12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
d) Stoffe mit physiologischer Kühlwirkung, vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), andere als in der vorliegenden Erfindung genannte Menthancarbonsäureamide (z.B. Menthancarbonsäure-N-ethylamid [WS3], Menthancarbonsäure-N-(p-methoxyphenyl)amid [SC1], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben), weitere Kühlwirkstoffe wie in WO2011061330 beschrieben, insbesondere Derivate verschieden substituierter Zimt- und 2-Phenoxysäuren, besonders bevorzugt Methylendioxyzimtsäure-N,N-diphenylamid, Methylendioxyzimtsäure-N-ethyl-N-phenylamid, Methylendioxyzimtsäure-N-pyridyl-N-phenylamid;
e) Stoffe mit adstringierender Wirkung, vorzugsweise ausgewählt aus der folgenden Liste: Catechine wie z.B. Epicatechine, Gallocatechine, Epigallocatechine sowie deren jeweiligen Gallussäureester, z.B. Epigallocatechingallat oder Epicatechingallat, dern Oligomere (Procyanidine, Proanthocyanidine, Prodelphinidine, Procyanirine, Thearubigenine, Theogalline) sowie deren C- und O-Glycoside; Dihydroflavonoide wie Dihydromyricetin, Taxifolin, sowie deren C- und O-Glycoside, Flavonole wie Myricetin, Quercetin sowie deren C- und O-Glycoside wie Quercetrin, Rutin, Gallussäaureester von Kohlenhydraten wie Tannin, Pentagalloylglucose oder deren Reaktionsprodukte wie Elligatannin, Aluminiumsalze, z.B. Alaun,

Insbesondere in den hierin beschriebenen erfindungsgemäß bzw. erfindungsgemäß bevorzugt zu verwendenden Konzentrationen haben Verbindungen der Formel (I) bzw. deren Salze oder Mischungen davon vorteilhafterweise häufig keine signifikanten anderen bzw. unerwünschten Aromawirkungen, können also besonders gut in vielen verschiedenen Aromatypen eingesetzt werden. Besonders vorteilhaft sind Aromakompositionen, die Kombinationen von Verbindungen der Formel (I) bzw. deren Salzen mit einem oder mehreren anderen trigeminal (scharf, wärmend, stechend, beißend, kratzend, kühlend, betäubend, kribbelnd (tingling), adstringierend) wirksamen Stoffen enthalten, wobei deren trigeminaler (Haupt-)Effekt durch Verbindungen der Formel (I) bzw. deren Salze vorteilhafterweise moduliert werden kann. Dabei kann beispielsweise ein wärmender, scharfer oder kühlender Effekt verstärkt werden, während ein adstringierender Effekt abgeschwächt werden kann.

Bevorzugt ist daher auch eine Aromakomposition, die zudem einen oder mehrere nicht der Formel (I) entsprechende Stoffe mit unangenehmer, insbesondere bitterer Geschmacksqualität, oder einer adstringierenden, bitteren, trockenen, staubigen, mehligen, kalkigen und/oder metallischen Note enthält, vorzugsweise ausgewählt aus der Gruppe bestehend aus:
f) Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallusoder Elagsäureester von Kohlenhydraten, z.B. Pentagalloylglucose, Tanninsäuren), nichthydrolisierbare Tannine (ggfs. galloylierte Catechine, Gallocatechine, Epigallocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. gamma-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside);
g) Stoffe mit einem nicht unangenehmen Primärgeschmack (beispielsweise süß, salzig, würzig, sauer) und/oder -geruch, vorzugsweise ausgewählt aus der Gruppe der Süßstoffe oder Zuckeraustauschstoffe, vorzugsweise Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Rebaudiosid A, Rebaudiosid B, Rebaudiosid C, Rebaudiosid D, Rebaudiosid X, Rubusoside, Hernandulcin, Thaumatin, Miraculin, Glycyrrhizin, Glycyrrhetinsäure, Balansin A oder Balansin B, oder deren Derivate, Cyclamat oder die pharmazeutisch akzeptablen Salze der vorgenannten Verbindungen.

Die vorliegende Erfindung betrifft weiterhin eine Pharmazeutische Zubereitung, der Ernährung, der Mundhygiene oder dem Genuss dienende Zubereitung, umfassend
(A) eine neue, erfindungsgemäße Mischung wie oben beschrieben,
   vorzugsweise wobei
   die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
   oder
(B) eine Verbindung der Formel (I), wie oben im Zusammenhang mit einer erfindungsgemäßen Verwendung definiert, oder ein physiologisch akzeptables Salz davon, wie oben im Zusammenhang mit einer erfindungsgemäßen Verwendung definiert, oder eine Mischung wie oben im Zusammenhang mit einer erfindungsgemäßen Verwendung definiert,
   wobei
   die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
   oder
(C) eine Aromakomposition wie oben beschrieben,
   vorzugsweise wobei
   die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg

Bevorzugt umfasst eine erfindungsgemäße Zubereitung zudem
ein oder mehrere übliche Grund-, Hilfs- und Zusatzstoffe in einer Menge von, bezogen auf das Gesamtgewicht der Zubereitung, 5 bis 99,999999 Gew.-%, vorzugsweise 10 bis 80 Gew.-%,
und/oder
Wasser in einer Menge, bezogen auf das Gesamtgewicht der Zubereitung, bis zu 99,999999 Gew.-%, vorzugsweise in einer Menge von 5 bis 80 Gew.-%.

Erfindungsgemäß bevorzugt ist auch eine Zubereitung wie oben beschrieben, wobei die Gesamtmenge an Verbindung(en) der Formel (I) und/oder Salz(en) davon in der Zubereitung ausreicht, um
(a) bei Gebrauch oder Verzehr der Zubereitung sensorisch einen wärmenden und/oder scharfen Effekt auf der Zunge oder im Mundraum hervorzurufen,
   und/oder
(b) einen unangenehmen Geschmackseindruck, vorzugsweise eines anderen in der Zubereitung enthaltenen Stoffes, insbesondere einen Geschmackseindruck ausgewählt aus der Gruppe bestehend aus adstringierend, bitter, trocken, staubig, mehlig, kalkig und metallisch, zu vermindern oder zu maskieren (vgl. hierzu oben),
   und/oder
(c) einen angenehmen Geschmackseindruck, vorzugsweise eines anderen in der Zubereitung enthaltenen Stoffes, insbesondere eines Geschmackseindrucks ausgewählt aus der Gruppe bestehend aus wärmend, scharf und kühlend, zu verstärken (vgl. hierzu oben).

Bevorzugt ist ferner eine erfindungsgemäße Zubereitung, die neben Verbindungen der Formel (I) bzw. deren Salzen (wie oben definiert) zumindest eine weitere Substanz zum Verändern, Maskieren oder Vermindern des unangenehmen Geschmackseindrucks eines unangenehm schmeckenden Stoffes oder Stoffgemisches umfasst. Entsprechend liegt dann eine Kombination von zumindest zwei Geschmackskorrigenzien vor.

Der Ernährung oder dem Genuss dienende Zubereitungen im Sinne der Erfindung sind z.B. Backwaren (z.B. Brot, Trockenkekse, Kuchen, sonstiges Gebäck), Süßwaren (z.B. Schokola-den, Schokoladenriegelprodukte, sonstige Riegelprodukte, Fruchtgummi, Hart- und Weichka-ramellen, Kaugummi), alkoholische oder nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Roibos-Tee, andere Kräutertee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obstund Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Pro-dukte, isoliertes oder enzymatisch behandeltes Sojaprotein enthaltende Getränke, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitung zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen.

Pharmazeutische Zubereitungen umfassen einen pharmazeutischen Wirkstoff. Vorteilhafte pharmazeutische Wirkstoffe sind beispielsweise steroidale entzündungshemmende Substanzen vom Kortikosteroiden-Typ wie z. B. Hydrocortison, Hydrocortison-Derivate wie Hydrocorti-son-17-butyrat, Dexamethason, Dexamethasonphosphat, Methylprednisolon oder Cortison. Vorteilhafte nichtsteroidale pharmazeutische Wirkstoffe sind beispielsweise Entzündungs-hemmer wie Oxicame wie Piroxicam oder Tenoxicam; Salicylate wie Aspirin® (Acetylsalicyl-säure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapro-pazon.

Besonders bevorzugte pharmazeutische Zubereitungen sind nicht verschreibungspflichtige Produkte und frei verkäufliche Arzneimittel, sogenannte OTC ("over the counter") -Präparate, enthaltend Wirkstoffe wie Paracetamol, Acetylsalicylsäure oder Ibuprofen, Vitamine (bei-spielsweise Vitamin H, Vitamine aus der B-Reihe wie Vitamin B1, B2, B6, B12, Niacin, Panthotensäure, vorzugsweise in Form von (Brause-)Tabletten oder Kapseln), Mineralien (vorzugsweise in Form von (Brause-)Tabletten oder Kapseln) wie Eisensalze, Zinksalze, Selensalze, Produkte enthaltend Wirkstoffe oder Extrakte von Spitzwegerich (z.B. in Hustensirup) oder Johanniskraut.

Die Zubereitungen im Sinne der Erfindung, welche auch unangenehm schmeckende Stoffe oder Stoffgemische enthalten können (vgl. hierzu oben), können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen sowie als Zubereitung mit funktionellen Inhaltsstoffen, als Nahrungsergänzungsmittel oder als bilanzierte Diäten.

Der Mundpflege dienende Zubereitungen im Sinne der Erfindung sind insbesondere Mund- und/oder Zahnpflegemittel wie Zahnpasten, Zahngele, Zahnpulver, Mundwässer, Kaugummis und andere Mundpflegemittel.

Zahnpflegemittel (als Basis für die Mundpflege dienende Zubereitungen), umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthal-temitteln wie z.B. Glycerin und/oder Sorbit, Verdickungsmittel, wie z.B. Carboxymethylcellulo-se, Polyethylenglycole, Carrageenan und/oder Laponite®, Süßstoffe, wie z.B. Saccharin, andere Geschmackskorrigenzien für unangenehme Geschmackseindrücke, Geschmackskorrigenzien für weitere, in der Regel nicht unangenehme Geschmackseindrücke, geschmacks-modulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Methylendioxyzimtsäure-N,N-diphenylamid, Methylendioxyzimtsäure-N-ethyl-N-phenylamid, Methylendioxyzimtsäure-N-pyridyl-N-phenylamid, Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigenzien.

Kaugummis (als weiteres Beispiel für die Mundpflege dienende Zubereitungen), umfassen im allgemeinen eine Kaugummibase, d.h. eine beim Kauen plastisch werdende Kaumasse, Zucker verschie-dener Arten, Zuckeraustauschstoffe, Süßstoffe, Zuckeralkoholen, andere Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), die im vorherigen Abschnitt genannten Kühlwirkstoffe, Feuchthaltemittel, Verdicker, Emulgatoren, Aromen und Stabilisatoren oder Geruchskorrigenzien.

Beispiele üblicher Grund-, Hilfs- und Zusatzstoffe für erfindungsgemäße Zubereitungen sind Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, andere als die erfindungsgemäß verwendeten Geschmackskorrigenzien für unangenehme Geschmackseindrücke (z. B. Hesperetin, Phloretin oder andere gemäß US 2008/0227867 zu verwendende Hydroxychalkonderivate sowie gegebenenfalls die dort beschriebenen Lactone), Geschmackskorrigenzien für weitere, in der Regel nicht unan-genehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure, Milchsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam, Stevioside, Rebaudioside, Acesulfam K, Neohesperidindihydrochalkon, Thaumatin, Superaspartam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

Die vorliegende Erfindung betrifft auch ein Verfahren zum Herstellen einer pharmazeutischen Zubereitung, einer der Ernährung, der Mundhygiene oder dem Genuss dienenden Zubereitung, vorzugsweise einer Zubereitung nach einem der Ansprüche 13 bis 15, umfassend folgende Schritte:
i) Bereitstellen
   (A) einer Mischung nach Anspruch 8 oder 9,
      vorzugsweise wobei
      die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
      oder
   (B) einer Verbindung der Formel (I), wie in einem der Ansprüche 1, 3, 4 oder 5 definiert, oder eines physiologisch akzeptablen Salz davon, wie in einem der Ansprüche 1, 3, 4 oder 5 definiert, oder einer Mischung wie in einem der Ansprüche 1, 3, 4 oder 5 definiert,
      wobei
      die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
      oder
   (C) eine Aromakomposition nach Anspruch 10, 11 oder 12,
      vorzugsweise wobei
      die Gesamtmenge an Verbindung(en) der Formel (I) und/oder Salz(en) so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
ii) Bereitstellen eines oder mehrerer weiterer Bestandteile der herzustellenden Zubereitung, und
iii) Inkontaktbringen oder Mischen der in Schritt ii) bereitgestellten weiteren Bestandteile mit dem/den in Schritt i) bereitgestellten Bestandteil(en), vorzugsweise in einer sensorisch wirksamen Menge.

Die beschriebenen erfindungsgemäßen Zubereitungen werden vorzugsweise hergestellt, indem ein erfindungsgemäß einzusetzender Ester der Homovanillinsäure als Substanz, als Lösung oder in Form einer Aromakomposition in eine der Ernährung, der Mundpflege oder dem Genuss dienende oder orale pharmazeutische Basis-Zubereitung eingearbeitet werden. Vorteilhafterweise können als Lösung vorliegende erfindungsgemäße Zubereitungen auch z.B. durch Sprühtrocknung in eine feste Zubereitung überführt werden.

Im Folgenden ist exemplarisch die Herstellung von erfindungsgemäßen Aromakompositionen (hier: enthaltend Ethylhomovanillat (**17**)) beschrieben, indem man
i) 1 Äquivalent Ascorbinsäure oder eines ihrer physiologisch akzeptablen Salze mit
ii) 1 Äquivalent Vanillylalkohol
iii) in einer (50/50; v/v) Mischung aus Wasser und einem Vertreter aus der Stoffgruppe der Alkohole, besonders bevorzugt sind Ethanol, Propanol, Butanol, Pentanol, Hexanol, Isopropylalkohol
iv) bei einer Temperatur von 100-150 °C (ggf. unter Druck)
v) für eine Zeit von 4-6 h
zur Reaktion bringt. Diese Aromazubereitung (primäres Reaktionsgemisch) enthält vorzugsweise 1.000-200.000 ppm, bevorzugt 10.000-100.000 ppm Ethylhomovanillat (**17**) und kann als solche oder gegebenenfalls weiter aufgereinigt in Mischung mit anderen Aromastoffen und Trägern als Aromakomposition verwendet werden. Diese Aromakompositionen enthalten vorzugsweise 500-100.000 mg/kg, bevorzugt 1.000-40.000 mg/kg besonders bevorzugt 1.000-15.000 mg/kg Ethylhomovanillat (**17**) oder physiologisch akzeptable Salze, insbesondere seine Natrium-, Kalium-, Ammonium-, Calcium-, Magnesium- oder Zinksalze, wobei die Konzentration von Ethylhomovanillat (**17**) bzw. Mischungen von Ethylhomovanillat (**17**) mit den entsprechenden Salzen im Endlebensmittel vorzugsweise 1-1.000 mg/kg, bevorzugt 1-750 mg/kg besonders bevorzugt 5-500 mg/kg entspricht.

Ascorbinsäure und Vanillylalkohol kommen jeweils in der Natur in Nahrungsmitteln vor und sind als Lebensmittelzusatzstoffe bzw. Aromastoffe zugelassen; besonders vorteilhaft ist daher der Einsatz von isolierter oder natürlich gewonnener Ascorbinsäure sowie von isoliertem oder natürlich gewonnenem Vanillylalkohol, wobei diese auch in Form von nicht vollständig aufgereinigten Extrakten oder Fraktionen eingesetzt werden können. Vanillylalkohol kommt z.B. in Bier (Flavor-Base, 9th Edition, Leffingwell & Associates, 2013) oder der Sitka-Fichte (*Picea sitchensis,* P. J. Kohlbrenner, C. Schuerch, Benzene-Alcohol-Soluble Extractives of Sitka Spruce, J. Org. Chem. 1959, 24(2), 166-172) vor.

Dabei sind die vorstehend beschriebenen erfindungsgemäßen Aromazubereitungen dadurch charakterisiert, dass sie neben Ethylhomovanillat (**17**) noch mindestens einen weiteren Stoff aus der folgenden Tab. 1 enthalten können (entsprechendes gilt für die hierin beschriebenen erfindungsgemäßen Aromakompositionen):

**Tab. 1:**

| **Nr.** | **Retentionszeit [min]*** | **Molare Masse m/z (Gef.: ESI+)** | **IPUAC Name** | **Struktur** |
|---|---|---|---|---|
| 1 | 2.88 | gef. 227.0546 ber. 226.047189 für C₁₀H₁₀O₆ | 3-(1,2-Dihydroxyethyl)-1,3-dihydroiso-benzofuran-1,4,5-triol | 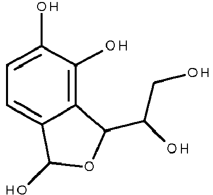 |
| 2 | 3.73 | gef. 313.0921 ber. 312.083969 für O₁₄H₁₆O₈ | 6-[(3-Hydroperoxy-4-hydroxy-phenyl)-methyl]-3,6,6a-trihydroxy-3,3a-dihydro-2H-furo[3,2-b]furan-5-on* | 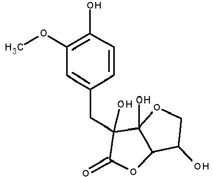 |
| 3 | 3.98 | gef. 313.0920 ber. 312.083969 für C₁₄H₁₆O₈ | 2-Hydroxy-3-(4-hydroxy-3-methoxy-phenyl)-2-(3-hydroxy-2-oxo-tetrahydrofuran-3-yl)propansäure | 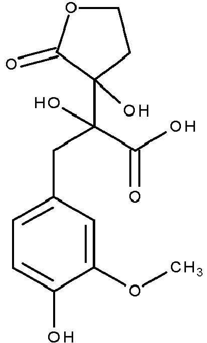 |
| 4 | 4.66 | gef. 251.0924 ber. 250.083575 für C₁₃H₁₄O₅ | 2-Hydroxy-3-(4-hydroxy-3-methoxy-phenyl)-4-(hydroxymethyl)cyclopent-2-en-1-on* | 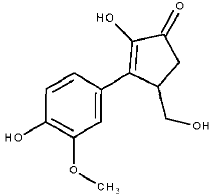 |
| 5 | 6.12 | gef. 387.1449 ber. 386.136004 für C₂₁H₂₂O₇ | 2-Hydroxy-3-[4-hydroxy-2-[(4-hydroxy-3-methoxy-phenyl)methyl]-5-methoxyphenyl]-4-(hydroxymethyl)cyclopent-2-en-1-on | 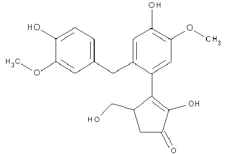 |
| 6 | 6.33 | gef. 251.0925 brr. 250.083575 für C₁₃H₁₄O₅ | 2-[(4-Hydroxy-3-methoxy-phenyl)-methylene]-5-(hydroxymethyl)-tetrahydrofuran-3-on | 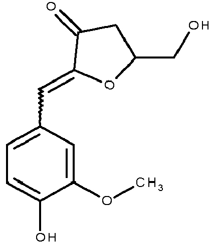 |
| 7 | 6.34 | gef. 251.0921 ber. 250.083575 für C₁₃H₁₄O₅ | 5-Hydroxy-6-[(4-hydroxy-3-methoxy-phenyl)methyl]-2,3-dihydropyran-4-on | 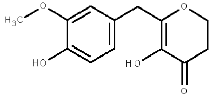 |
| 8 | 6.98 | gef. 341.1251 ber. 340.115269 für C₁₆H₂₀O₈ | Ethyl-2-hydroxy-3-(4-hydroxy-3-methoxy-phenyl)-2-(3-hydroxy-2-oxo-tetrahydro-furan-3-yl)propanoat | 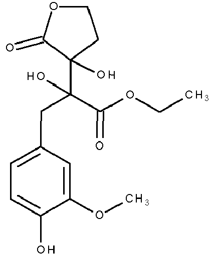 |
| 9 | 7.68 | gef. 233.0842 ber. 232.073010 für C₁₃H₁₂O₄ | 1-(2-Furyl)-2-(4-hydroxy-3-methoxy-phenyl)ethanon | 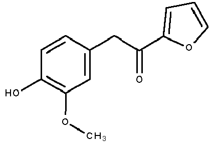 |
| 10 | 7.71 | gef. 387.1457 ber. 386.136004 für C₂₁H₂₂O₇ | 2-Hydroxy-3-[4-hydroxy-3-[(4-hydroxy-3-methoxy-phenyl)methyl]-5-methoxy-phenyl]-4-(hydroxymethyl)cyclopent-2-en-1-on | 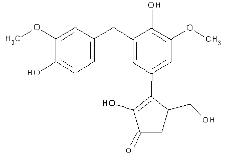 |
| 11 | 7.93 | gef. 233.0821 ber. 233.073010 für C₁₃H₁₂O₄ | 2-[(4-Hydroxy-3-methoxy-phenyl)methylene]-5-methyl-furan-3-on | 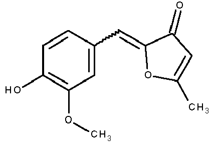 |
| 12 | 8.22 | gef. 297.1347 ber. 296.125440 für C₁₅H₂₀O₆ | Ethyl-3-[3-(4-hydroxy-3-methoxy-phenyl)-2-oxo-propoxy]propanoat | 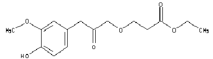 |
| 14 | 8.82 | gef. 465.1606 ber. 464.146569 für C₂₆H₂₄O₈ | 2-[3-(2-Furyl)-1,2-bis(4-hydroxy-3-methoxy-phenyl)-3-oxo-propyl]-5-methyl-furan-3-on | 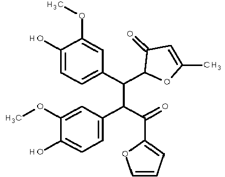 |
| 15 | 9.52 | gef. 369.1346 ber. 368.125440 für C₂₁H₂₀O₆ | 1-(2-Furyl)-2-[4-hydroxy-2-[(4-hydroxy-3-methoxy-phenyl)methyl]-5-methoxyphenyl]ethanon | 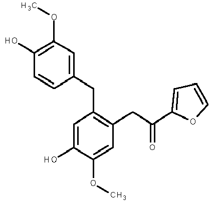 |

| | | | | |
|---|---|---|---|---|
| * s. Preobrazhenskaya, M.N. et al. Tetrahedron 1997, 53, 6971-6976 | | | | |

Die Aufreinigung des primären Reaktionsgemischs kann erfolgen durch eines oder mehrere der folgenden Verfahren:
a) gegebenenfalls Aufkonzentrierung des primären Reaktionsgemischs, vorzugsweise durch ein oder mehrere evaporative oder pervaporative Verfahren,
b) gegebenenfalls Behandlung des (gegebenenfalls in Schritt a) aufkonzentrierten) primären Reaktionsgemischs durch verteilungschromatographische (z.B. Gegenstromverteilungsverfahren wie FCPC, SCCC, Craig-Verfahren) oder adsorptionschromatographische Verfahren mit oder an Adsorbentien, vorzugsweise ausgewählt aus der Gruppe bestehend aus Kieselgel, modifiziertes Kieselgel, Aktivkohle, Zeolith, Bentonit, Kieselgur, Aluminiumoxid, basischer oder saurer oder neutraler, optional makroporöser, Ionentauscher, bevorzugt im Batch- oder Säulenverfahren, gegebenenfalls unter Zuhilfenahme weiterer Extraktionsmittel, wodurch eine aufgereinigte Aromakomposition erhalten wird,
c) gegebenenfalls Trocknung der in Schritt b) erhaltenen aufgereinigten Aromakomposition, vorzugsweise durch ein evaporatives oder pervaporatives Verfahren,
d) gegebenenfalls ein- oder mehrmaliger Wiederholung der Schritte b) und c)
e) gegebenenfalls Mischen der in den vorherigen Schritten erhaltenen aufgereinigte Aromakomposition mit einem geeigneten Verdünnungsmittel oder einem Gemisch zweier oder mehrerer Verdünnungsmittel, bevorzugt ausgewählt aus der Gruppe bestehend aus Ethanol, Isopropanol, 1,2-Propylenglycol, Pflanzenöltriglyceriden, Diacetin, Triacetin und Glycerin, wobei vorzugsweise die Aromakomposition in Form einer Lösung erhalten wird.

Gemäß einer weiteren bevorzugten Ausführungsform werden zur Herstellung erfindungsgemäßer Zubereitungen erfindungsgemäß verwendete Verbindungen der Formel (I) bzw. deren Salze oder eine erfindungsgemäße Aromakomposition, insbesondere das primäre Reaktionsgemisch (wie oben exemplarisch beschrieben) oder die aufgereinigten Aromakompositionen (wie oben exemplarisch beschrieben) und gegebenenfalls andere Bestandteile der erfindungsgemäßen Zubereitung in Form von Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet. In einem bevorzugten Herstellungsverfahren werden die Verbindungen der Formel (I) bzw. deren Salze vor dem Einarbeiten mit einem oder mehreren geeigneten Komplexbildnern, beispielsweise mit Cycloglycanen, z.B. Cyclofructanen, Cyclodextrinen oder Cyclo-dextrinderivaten, bevorzugt alpha-, beta- und gamma-Cyclodextrin, komplexiert und in dieser komplexierten Form eingesetzt.

Im Folgenden wird die vorliegende Erfindung anhand ausgewählter, konkreter Beispiele näher beschrieben. Die Beispiele dienen nur zur Verdeutlichung der Erfindung, ohne diese einzuschränken bzw. darauf zu beschränken. Sofern nicht anders angegeben, beziehen sich alle Angaben auf das Gewicht.

### Beispiele

### Beispiel 1: Herstellung einer Aromazubereitung (primäres Reaktionsgemisch) enthaltend Ethylhomovanillat (17) durch Reaktion von Ascorbinsäure mit Vanillylalkohol

3 mmol Ascorbinsäure und 3 mmol Vanillylalkohol wurden in 10 mL Wasser/Ethanol (1/1; v/v) gelöst. Die Lösung wurde unter stetigem Rühren in der Mikrowelle (Mars Synthesis, CEM) in 7 min auf 100°C erhitzt. Anschließend wurde das Reaktionsgemisch weitere 6 h bei 100°C unter stetigem Rühren in der Mikrowelle erhitzt. Im unten abgebildeten LC-MS/QTOF-Chromatogramm sind die nach 6 h entstehenden in Tabelle 1 aufgelisteten Substanzen sowie Ethylhomovanillat (EHV, **17**) zu sehen. Ethylhomovanillat (**17**) ist zu 1.2% in dem primären Reaktionsgemisch enthalten.

Die Durchführung der obigen Reaktion mit den aufgeführten Verhältnissen an Ascorbinsäure und Vanillylalkohol mit einer Kochzeit von 4 h führt ebenfalls zur Entstehung von EHV.

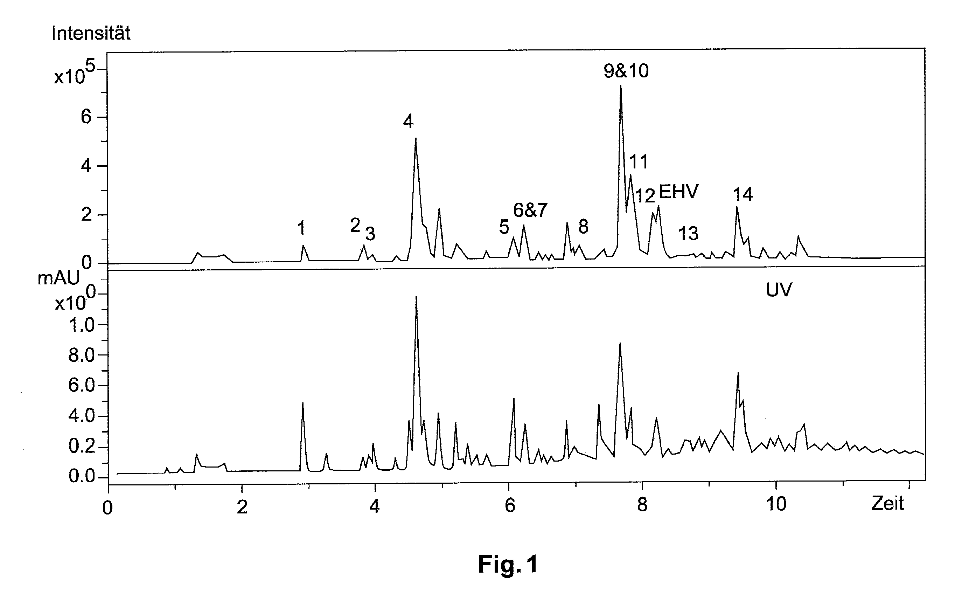

Siehe hierzu Fig. 1 (LC-MS/QTOF Chromatogramm des primäres Reaktionsgemischs nach 6 h bei 100°C; oberes Chromatogramm Massenspur ESI positiv, unteres Chromatogramm UV-VIS summiert; die Nummern stellen die Verbindungen nach Tabelle 1 dar und EHV steht für Ethylhomovanillat (**17**)).

### Beispiel 2: Isolierung von Ethylhomovanillat (17) aus einer Aromazubereitung (primäres Reaktionsgemisch)

Das primäre Reaktionsgemisch wird mittels Mitteldruckchromatographie (MPLC) (Säulenmaterial: Lewatit VP OC 1064; Wasser/Ethanol 3/1; v/v) vorfraktioniert. Anschließend erfolgt eine weitere Auftrennung via präparativer Hochdruckchromatographie (pHPLC) (Säule: Phenomenex Luna C18 5µ 150x21.2 mm; Flussrate 30mL/min; Detektion 210 nm) im isokratischen Modus (63% H₂O, 37% MeOH). Die abschließende Isolierung von Ethylhomovanillat (**17**) wird via semipräparativer Hochdruckchromatographie (sPHPLC) im Gradienten-Modus (Säule: YMC Triart C18 5µ 250x10mm; A: H₂O; B: MeOH; 0min 65% A, 35% B; 25min 40% A, 60% B; 30min 100% B; Flussrate 3 mL/min; Detektion: 250 nm) durchgeführt. Das erhaltene Ethylhomovanillat (**17**) wurde anschließend gefriergetrocknet und in einer Dosierung von 100 ppm auf 5%-ige Zucker-, 0.5%ige Salz-, 500 ppm Coffeinlösung und Wasser verkostet und sensorisch bewertet.

| **Medium** | **Geschmacksbeschreibung** |
|---|---|
| Wasser | scharf, stechend, leicht wärmend |
| 5%-ige Zuckerlösung | scharf, wärmend |
| 0.5%-ige Salzlösung | scharf |

### Beispiel 3: Herstellung und sensorische Evaluierung einer Fraktion (aufgereinigte Aromazubereitung) enthaltend Ethylhomovanillat (17)

Das primäre Reaktionsgemisch wird mittels LC-Taste^{®} (nach WO 2006 111,476) im Gradientenmodus (Hamilton PRP-1 10 µ 250x21.5 mm; A: H₂O, B: EtOH; 0 min 100% A; 25 min 75% A, 25% B; 40 min 100% B; Flussrate: 10 mL/min, Ofentemperatur: 80 °C) in 12 Fraktionen geschnitten, wobei die Fraktionseinteilung anhand der UV Spur bei 210 nm erfolgte. Nach Einengung der Fraktionen auf 0.5 mL am Büchi Syncore bei 40 °C wurde der Rückstand in 10 mL Wasser gelöst. Von diesen Lösungen wurden jeweils 2 mL mit 18 mL 3.33%iger Zuckerlösung vermischt (entspricht einer Enddosierung von 96 mg/kg Ethylhomovanillat (**17**) für Fraktion 10 und 26 mg/kg Ethylhomovanillat für Fraktion 11 in 3%iger Zuckerlösung) und sensorisch bewertet.

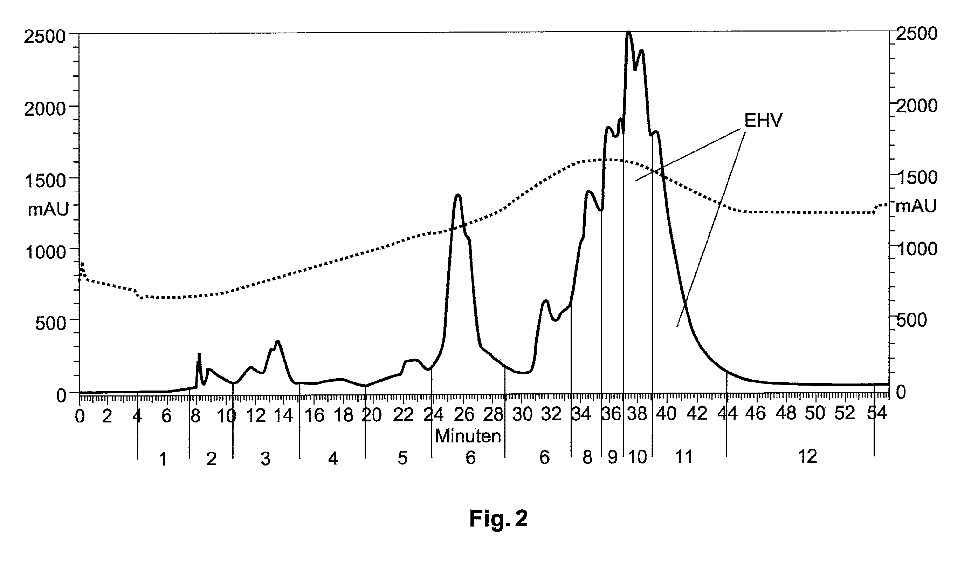

Siehe hierzu Fig. 2 (LC Taste Chromatogramm des primären Reaktionsgemischs; Fraktion 10 und 11 enthalten EHV)

**Tab. 2: Sensorisches Geschmacksprofil der Fraktionen 10 und 11 der LC Taste Trennung (siehe Fig. 2)**

| Fraktion | Geschmack | Intensität |
|---|---|---|
| 10 | wärmend | 5-6 |
| | medizinisch | 4-5 |
| | Paprika | 3-4 |
| | Geräucherter Schinken (rauchig) | 3 |
| | Scharf | 5 |
| 11 | wärmend | 2-3 |
| | geräucherter Schinken | 4-5 |
| | rauchig | 4 |
| | gegrillt, BBQ | 4 |
| | phenolisch | 3 |
| | Vanille | 2 |

### Beispiel 4: Synthese von Homovanillinsäureestern

### Methode A:

Homovanillinsäure (1.5-3 g) wurde mit dem jeweiligen Alkohol (äquimolar) in Toluol (100 mL) vorgelegt, konz. Schwefelsäure dazugegeben und für 5 h am Wasserabscheider zum Sieden erhitzt. Es wurde einmal mit ges. wäss. NaHCO₃-Lösung, zweimal mit Wasser oder wahlweise ges. wäss. NaCl-Lösung gewaschen und das Lösungsmittel im Vakuum entfernt. Das Produkt wurde nach säulenchromatographischer Reinigung an Kieselgel in ca. 70% Ausbeute erhalten.

### Methode B:

Homovanillinsäure (1.5-3 g) wurde in dem jeweiligen Alkohol (100 mL) und 0.2-0.5 Äquivalenten Schwefelsäure für 7 h bei 90 °C (Heizblocktemperatur) gerührt. Der größte Teil des Alkohols wurde im Vakuum entfernt, ges. wäss. NaHCO₃-Lösung und EtOAc dazugegeben, die org. Phase abgetrennt und die wäss. Phase einmal mit EtOAc extrahiert. Die vereinigten org. Phasen wurden je einmal mit ges. wäss. NaHCO₃- und mit Wasser oder wahlweise ges. wäss. NaCl-Lösung, über NaSO₄ getrocknet und das Lösungsmittel im Vakuum entfernt. Das Produkt wurde nach säulenchromatographischer Reinigung an Kieselgel in 90-quant. Ausbeute erhalten.

### Isopropyl-2-(4-hydroxy-3-methoxy-phenyl)acetat (7, Methode B)

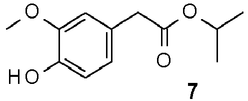

¹H-NMR (400 MHz, CDCl₃): *δ* = 6.85 (d, *J* = 8.1 Hz, 1 H), 6.81 (dd, *J* = 2.0, 0.5 Hz, 1 H), 6.76 (ddt, *J =* 8.0, 2.0, 0.6 H₂,1H), 5.60 (s, 1H), 5.01 (hept, *J* = 6.3 Hz, 1H), 3.88 (s, 3H), 3.496 (t, *J =* 0.5 Hz, 2H), 1.23 (d, *J =* 6.3 Hz, 6H).
¹³C-NMR (100 MHz, CDCl₃): *δ* = 171.5, 146.4, 144.7, 126.1, 122.1, 114.3, 111.7, 68.1, 55.9, 41.3, 21.8 (2C).
GCMS: *m*/*z* (%) = 224 [M⁺] (30), 137 (100), 122 (10), 107 (2), 94 (6), 77 (3), 66 (5), 51 (3), 43 (15).

### sec-Butyl-2-(4-hydroxy-3-methoxy-phenyl)acetat (8, Methode B)

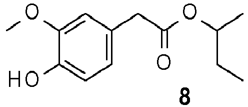

¹H-NMR (400 MHz, CDCl₃): *δ* = 6.85 (d, *J =* 8.1 Hz, 1 H), 6.82 (d, *J =* 1.9 Hz, 1 H), 6.79-6.74 (m, 1 H), 5.57 (s, 1 H), 4.92-4.78 (m, 1H), 3.88 (s, 3H), 3.51 (t, *J* = 0.5 Hz, 2H), 1.62-1.46 (m, 2H), 1.19 (d, *J =* 6.3 Hz, 3H), 0.85 (t, *J* = 7.5 Hz, 3H).
¹³C-NMR (100 MHz, CDCl₃): *δ* = 171.6, 146.4, 144.7, 126.2, 122.1, 114.3, 111.7, 72.7, 55.9, 41.4, 28.8, 19.4, 9.6.
GCMS: *m*/*z* (%) = 238 [M⁺] (30), 137 (100), 122 (8), 107 (2), 94 (5), 77 (2), 66 (3), 57 (20), 51 (2), 41 (8), 29 (8).

### Isobutyl-2-(4-hydroxy-3-methoxy-phenyl)acetate (9, Methode B)

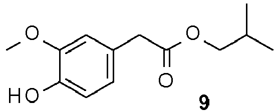

¹H-NMR (400 MHz, CDCl₃): *δ* = 6.85 (dd, *J* = 8.1, 0.3 Hz, 1 H), 6.82-6.81 (m, 1 H), 6.77 (ddt, *J* = 8.1, 2.0, 0.6 Hz, 1 H), 5.60 (s, 1 H), 3.87 (d, *J* = 0.3 Hz, 3H), 3.86 (d, *J* = 6.6 Hz, 2H), 3.54 (t, *J* = 0.5 Hz, 2H), 1.91 (dq, *J* = 6.7 Hz, 1 H), 0.90 (d, *J* = 6.7 Hz, 6H).
¹³C-NMR (100 MHz, CDCl₃): *δ* = 172.0, 146.5, 144.7, 126.0, 122.1, 114.3, 111.7, 70.9, 55.9, 41.1, 27.7, 19.0 (2C).
GCMS: *m*/*z* (%) = 238 [M⁺] (30), 182 (5), 137 (100), 122 (9), 107 (2), 94 (6), 77 (2), 66 (3), 57 (11), 51 (2), 41 (8), 29 (7).

### Butyl-2-(4-hydroxy-3-methoxy-phenyl)acetat (19, Methode B)

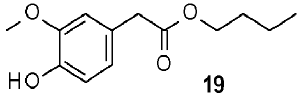

¹H-NMR (400 MHz, CDCl₃): *δ* = 6.85 (d, *J* = 8.1 Hz, 1 H), 6.81 (dd, *J* = 2.0, 0.5 Hz, 1 H), 6.76 (ddt, *J =* 8.1, 1.9, 0.6 Hz, 1H), 5.60 (s, 1 H), 4.09 (t, *J =* 6.7 Hz, 2H), 3.87 (d, *J* = 0.3 Hz, 3H), 3.53 (t, *J =* 0.5 Hz, 2H), 1.67-1.55 (m, 2H), 1.42-1.29 (m, 2H), 0.91 (t, *J =* 7.4 Hz, 3H).
¹³C-NMR (100 MHz, CDCl₃): *δ* = 172.0, 146.5, 144.7, 126.0, 122.1, 114.3, 111.7, 64.7, 55.9, 41.1, 30.6, 19.1, 13.7.
GCMS: *m*/*z* (%) = 238 [M⁺] (27), 182 (2), 137 (100), 122 (9), 107 (2), 94 (5), 77 (2), 66 (2), 57 (4), 41 (5), 29 (8).

### Propyl-2-(4-hydroxy-3-methoxy-phenyl)acetat (18, Methode B)

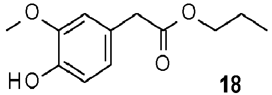

¹H-NMR (400 MHz, CDCl₃): *δ* = 6.85 (d, *J* = 8.0 Hz, 1 H), 6.81 (d, *J* = 2.0 Hz, 1 H), 6.76 (dd, *J* = 8.1, 2.0 Hz, 1 H), 5.61-5.58 (m, 1 H), 4.05 (t, *J =* 6.7 Hz, 2H), 3.88 (s, 3H), 3.54 (s, 2H), 1.71-1.52 (m, 2H), 0.91 (t, *J =* 7.4 Hz, 3H).
¹³C-NMR (100 MHz, CDCl₃): *δ* = 172.0, 146.5, 144.7, 126.0, 122.2, 144.3, 111.7, 66.4, 55.9, 41.1, 22.0, 10.4.
GCMS: *m*/*z* (%) = 224 [M⁺] (30), 137 (100), 122 (10), 107 (2), 94 (8), 77 (2), 66 (3), 51 (2) 43 (8).

### Ethyl-2-(4-hydroxy-3-methoxy-phenyl)acetat (17, Methode B)

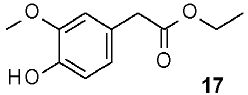

¹H-NMR (400 MHz, CDCl₃): *δ* = 6.85 (d, *J* = 8.05 Hz, 1H), 6.81 (d, *J* = 1.94 Hz, 1H), 6.76 (ddd, *J =* 8.01, 1.99, 0.50 Hz, 1H), 5.61 (d, *J* = 0.36 Hz, 1 H), 4.15 (q, *J* = 7.13 Hz, 2H), 3.88 (s, 3H), 3.53 (d, *J =* 0.57 Hz, 2H), 1.25 (t, *J* = 7.13 Hz, 1 H).
¹³C-NMR (100 MHz, CDCl₃): *δ* = 172.0, 146.5, 144.7, 125.9, 122.1, 114.4, 111.7, 60.8, 55.9, 41.0, 14.2.
GCMS: *m*/*z* (%) = 210 [M⁺] (30), 137 (100), 122 (11), 107 (2), 94 (8), 77 (2), 66 (3), 51 (3), 39 (3), 29 (8).

### Hexyl-2-(4-hydroxy-3-methoxy-phenyl)acetat (21, Methode A)

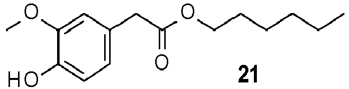

¹H-NMR (400 MHz, CDCl₃): *δ* = 6.85 (dd, *J* = 8.1, 0.3 Hz, 1 H), 6.81 (d, *J* = 2.0 Hz, 1 H), 6.76 (ddq, *J =* 8.1, 2.0, 0.5 Hz, 1H), 5.58 (s, 1H), 4.08 (t, *J =* 6.7 Hz, 2H), 3.88 (s, 3H), 3.53 (s, 2H), 1.65-1.56 (m, 2H), 1.36-1.20 (m, 6H), 0.87 (t, *J =* 7.0 Hz, 3H).
¹³C-NMR (100 MHz, CDCl₃): *δ* = 172.0, 146.5, 144.7, 126.0, 122.1, 114.3, 111.7, 65.0, 55.9, 41.1, 31.4, 28.6, 25.5, 22.5, 14.0.
GCMS: *m*/*z* (%) = 266 [M⁺] (30), 182 (8), 137 (100), 122 (8), 107 (2), 94 (4), 77 (2), 66 (2), 55 (3), 43 (13).

### [(Z)-Hex-3-enyl]-2-(4-hydroxy-3-methoxy-phenyl)acetat (6, Methode A)

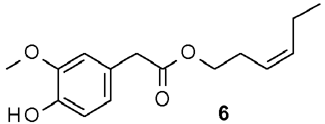

¹H-NMR (400 MHz, CDCl₃): *δ* = 6.85 (d, *J* = 8.1 Hz, 1 H), 6.80 (d, *J* = 2.0 Hz, 1 H), 6.76 (dd, *J* = 8.0, 2.0 Hz, 1H), 5.58 (s, 1H), 5.49 (ddt, *J* = 10.9, 7.3, 1.6 Hz, 1 H), 5.29 (ddt, *J* = 10.7, 7.3, 1.5 Hz, 1 H), 4.08 (t, *J* = 7.0 Hz, 2H), 3.88 (s, 3H), 3.53 (s, 2H), 2.43-2.32 (m, 2H), 2.03 (pd, *J* = 7.5, 1.6 Hz, 2H), 0.96 (t, *J* = 7.5 Hz, 3H).
¹³C-NMR (100 MHz, CDCl₃): *δ* = 171.9, 146.5, 144.7, 134.6, 125.8, 123.6, 122.1, 114.3, 111.7, 64.4, 55.9, 41.0, 26.7, 20.6, 14.2.
GCMS: *m*/*z* (%) = 264 [M⁺] (30), 182 (55), 137 (100), 122 (15), 94 (10), 82 (8), 67 (15), 55 (20), 41 (15).

### 2-Methylbutyl-2-(4-hydroxy-3-methoxy-phenyl)acetat (12, Methode B)

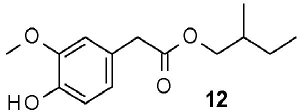

¹H-NMR (400 MHz, CDCl₃): *δ* = 6.86 (d, *J* = 8.1 Hz, 1 H), 6.81 (d, *J* = 2.0 Hz, 1 H), 6.77 (dd, *J* = 8.0, 2.0 Hz, 1H), 5.55 (s, 1H), 3.97 (dd, *J* = 10.7, 6.0 Hz, 1H), 3.90 (dd, *J* = 10.8, 6.7 Hz, 1H), 3.88 (s, 3H), 3.54 (s, 2H), 1.69 (dddd, *J* = 12.4, 7.8, 6.8, 5.8 Hz, 1H), 1.38 (dtd, *J* = 13.1, 7.5, 5.6 Hz, 1H), 1.23-1.08 (m, 1 H), 0.88 (d, *J* = 6.8 Hz, 3H), 0.88 (t, *J* = 7.5 Hz, 3H).
¹³C-NMR (100 MHz, CDCl₃): *δ* = 172.0, 146.4, 144.7, 126.0, 122.1, 114.3, 111.7, 69.4, 55.9, 41.1, 34.1, 26.0, 16.3, 11.2.
GCMS: *m*/*z* (%) = 252 [M+] (30), 182 (10), 137 (100), 122 (8), 94 (7), 71 (5), 55 (4), 43 (18), 29 (10).

### 2-Phenylethyl-2-(4-hydroxy-3-methoxy-phenyl)acetat (1, Methode A)

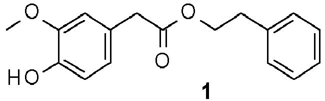

¹H-NMR (400 MHz, CDCl₃): *δ* = 7.29-7.24 (m, 2H), 7.24-7.19 (m, 1H), 7.16 - 7.12 (m, 2H), 6.85 (d, *J* = 7.9 Hz, 1 H), 6.74 (d, *J =* 1.9 Hz, 1 H), 6.72 (dd, *J* = 8.0, 1.9 Hz, 1 H), 5.57 (s, 1 H), 4.30 (t, *J =* 6.9 Hz, 2H), 3.84 (s, 3H), 3.51 (s, 2H), 2.91 (t, *J =* 6.9 Hz, 2H).
¹³C-NMR (100 MHz, CDCl₃): *δ* = 171.8, 146.4, 144.7, 137.7, 128.9 (2C), 128.4 (2C), 126.5, 125.7, 122.2, 114.3, 111.7, 65.3, 55.87, 41.1, 35.0.
GCMS: *m*/*z* (%) = 286 [M⁺] (30), 182 (48), 137 (100), 122 (12), 105 (30), 94 (11), 77 (12), 65 (8), 51 (7), 39 (5).

### Pentyl-2-(4-hydroxy-3-methoxy-phenyl)acetat (20, Methode A)

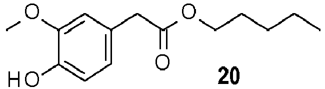

¹H-NMR (400 MHz, CDCl₃): *δ* = 6.85 (d, *J* = 8.0 Hz, 1 H), 6.81 (d, *J* = 2.0 Hz, 1 H), 6.76 (dd, *J* = 8.1, 2.0 Hz, 1H), 5.58 (s, 1H), 4.08 (t, *J* = 6.7 Hz, 2H), 3.88 (s, 3H), 3.53 (s, 2H), 1.61 (q, *J* = 6.7 Hz, 2H), 1.37-1.24 (m, 4H), 0.94-0.83 (m, 3H).
¹³C-NMR (100 MHz, CDCl₃): *δ* = 172.0, 146.5, 144.7, 126.0, 122.1, 114.3, 111.7, 65.0, 55.9, 41.1, 28.3, 28.0, 22.3, 14.0.
GCMS: *m*/*z* (%) = 252 [M⁺] (28), 182 (5), 137 (100), 122 (10), 94 (5), 66 (3), 43 (13), 29 (4).

### Heptyl-2-(4-hydroxy-3-methoxy-phenyl)acetat (14, Methode A)

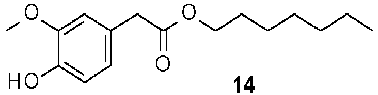

¹H-NMR (400 MHz, CDCl₃): *δ* = 6.85 (dd, *J* = 8.1, 1.1 Hz, 1H), 6.81 (t, *J* = 1.4 Hz, 1H), 6.76 (dt, *J =* 8.2, 1.4 Hz, 1 H), 5.59 (d, *J =* 1.1 Hz, 1 H), 4.08 (td, *J* = 6.8, 1.1 Hz, 2H), 3.88 (d, *J =* 1.2 Hz, 3H), 3.53 (s, 2H), 1.68-1.55 (m, 2H), 1.28 (td, *J* = 9.8, 9.3, 4.0 Hz, 8H), 0.94-0.82 (m, 3H).
¹³C-NMR (100 MHz, CDCl₃): *δ* = 172.0, 146.5, 144.7, 126.0, 122.1, 114.3, 111.7, 65.0, 55.9, 41.1, 31.7, 28.9, 28.6, 25.8, 22.6, 14.1.
GCMS: *m*/*z* (%) = 280 [M⁺] (34), 182 (12), 137 (100), 122 (8), 94 (5), 57 (11), 41 (8).

### Beispiel 5: Verkostung von Homovanillinsäureestern

Die zu verkostende Substanz wurde in Ethanol gelöst und die ethanolische Lösung dann mit 5%iger Zuckerlosung verdünnt (Endkonzentration: 25 ppm). Zur Verkostung wurde von 4 Prüfern der Mundraum jeweils mit ca. 5 mL der Zuckerlösung gespült und die Lösung wieder ausgespuckt. Die Schärfe wurde auf einer Skala 1 (sehr schwach) - 9 (sehr stark) eingeschätzt und das Profil bewertet.
a) Profil Hexyl-2-(4-hydroxy-3-methoxy-phenyl)acetat (**21**): Deutlich scharf, leicht verzögerter Effekt, wärmend, relativ schnelle Abnahme der Schärfe; Einschätzung der Schärfe 9.
b) Profil Butyl-2-(4-hydroxy-3-methoxy-phenyl)acetat (**19**): Unmittelbare Schärfe, schnelle Abnahme; Einschätzung der Schärfe: 7.
c) Profil Isobutyl-2-(4-hydroxy-3-methoxy-phenyl)acetat (**9**): Schnelles Einsetzen der Schärfe, wärmend, etwas beißende Schärfe; Einschätzung der Schärfe: 4-5.
d) Profil Propyl-2-(4-hydroxy-3-methoxy-phenyl)acetat (**18**): Schwache und verzögerte Schärfe, sich aufbauend wärmend, Tingling; Einschätzung der Schärfe: 4-5.
e) Profil Ethyl-2-(4-hydroxy-3-methoxy-phenyl)acetat (**17**): Etwas wärmend, etwas scharf; Einschätzung der Schärfe: 1-2.
f) Profil Isopropyl 2-(4-hydroxy-3-methoxy-phenyl)acetat (**7**): Spätes Einsetzen, Tingling, wärmend, scharf, etwas verzögert, nicht langlebig, angenehm; Einschätzung der Schärfe: 3.
g) Profil *sec*-Butyl-2-(4-hydroxy-3-methoxy-phenyl)acetat (**8**): sehr langsames Einsetzen, Tingling, hauptsächlich wärmend, Schärfe sich aufbauend, Einschätzung der Schärfe: 3.
h) Profil Octyl-2-(4-hydroxy-3-methoxy-phenyl)acetat (nicht erfindungsgemäß): Schärfe setzt langsam ein, spät, verzögert, fettig-fruchtige Nebennote, brennend; Einschätzung der Schärfe: 7.
i) Profil Decyl-2-(4-hydroxy-3-methoxy-phenyl)acetat (nicht erfindungsgemäß): Schwache Schärfe, verzögert, Birnen-artig und fettig-fruchtige Nebennote, brennend mehr im Hals als auf der Zunge; Einschätzung der Schärfe: 2.

### Beispiel 6: Isointensität von Homovanillinsäureestern im Vergleich zu Nonivamid und einem Capsicum-Extrakt

Die zu verkostende Substanz wurde in Ethanol gelöst und die ethanolische Lösung dann mit 5%iger Zuckerlosung verdünnt (Endkonzentration: 10 ppm). Zum Vergleich wurden Capsicum-Extrakt mit 1.000.000 SHU (0.3-10 ppm) und Nonivamid (0.1-1 ppm) in 5%iger Zuckerlösung in aufsteigender Konzentration vorbereitet. Zur Verkostung wurde von 4 Prüfern der Mundraum jeweils mit ca. 5 mL der Verkostungslösung gespült und die Lösung wieder ausgespuckt und gegen die Referenzreihen bewertet.

Die Schärfe von 10 ppm Hexyl-2-(4-hydroxy-3-methoxy-phenyl)acetat (**21**) ist vergleichbar mit der von 0.5 ppm Nonivamid.

Die Schärfe von 10 ppm Butyl-2-(4-hydroxy-3-methoxy-phenyl)acetat (**19**) ist vergleichbar mit der von 0.3 ppm Nonivamid.

Die Schärfe von 10 ppm Butyl-2-(4-hydroxy-3-methoxy-phenyl)acetat (**19**) ist vergleichbar mit der von 4.5 ppm Capsicum-Extrakt 1.000.000 SHU.

Die Schärfe von 10 ppm Hexyl-2-(4-hydroxy-3-methoxy-phenyl)acetat (**21**) ist vergleichbar mit der von 8.5 ppm Capsicum-Extrakt 1.000.000 SHU.

### Beispiel 7: Schwellenwerte von Homovanillinsäureestern

Die Schwellenwerte wurden nach der Methode ASTM E 679 - 91 ("Standard Practice for Determination of Odor and Taste Thresholds By a Forced-Choice Ascending Concentration Series Method of Limits1 ") bestimmt. Es handelt sich um die jeweilige Flavor Reizschwelle auf Vittel^{®} Wasser.

Zum Beispiel der Schwellenwert von Hexyl-2-(4-hydroxy-3-methoxy-phenyl)acetat (**21**) in Wasser liegt bei 1.7 ppm (1700 ppb).

### Beispiel 8: Schärfeprofil von Butyl-2-(4-hydroxy-3-methoxy-phenyl)acetat (19) im Vergleich zu Nonivamid

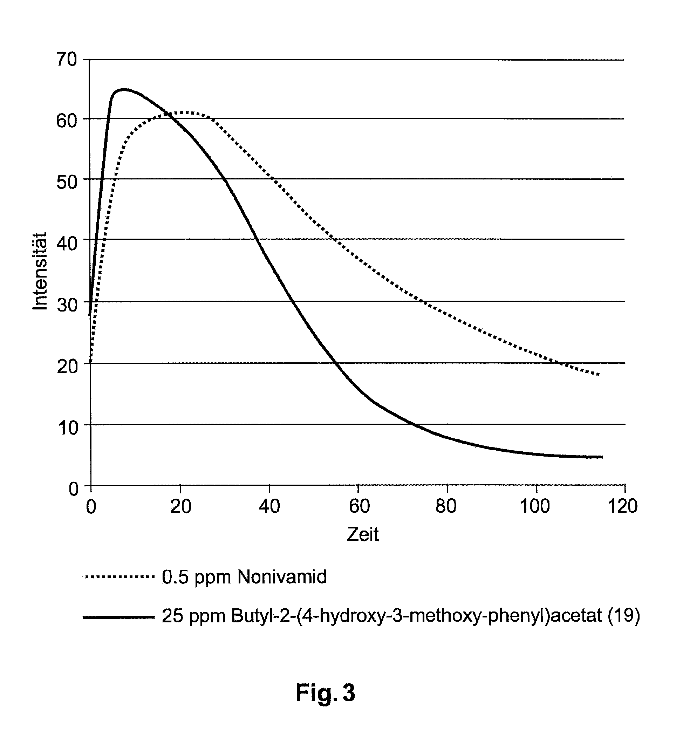

Zur Erstellung eines Zeit-Intensitäts-Profils wurde der Mundraum von geschulten Panelisten (n = 8-10) mit einem Schluck einer Probenlösung (5 mL einer 5% Zuckerlösung) gespült. Dann wurde die Intensität der Eigenschaft Schärfe in definierten Zeitabständen anhand einer Skala ohne feste Einteilung bewertet. Die zwei Verkostungslösungen (Butyl-2-(4-hydroxy-3-methoxy-phenyl)acetat (**19**) mit 25 ppm und Nonivamid mit 0.5 ppm der entsprechenden Substanz wurden dabei codiert und in gemischter Reihung platziert. Zwischen den beiden Verkostungsproben mit Brot und Wasser wieder neutralisiert und mit 5 mL 5%ige Zuckerlösung vorgespült. Die Daten wurden analysiert und graphisch in einer Zeit-Intensitätskurve wiedergegeben (s. Fig. 3 (Schärfeprofil Butyl-2-(4-hydroxy-3-methoxyphenyl)acetat (**19**) im Vergleich zu Nonivamid)).

Deutlich zu erkennen ist das schnellere Einsetzen der Schärfe von Butyl-2-(4-hydroxy-3-methoxyphenyl)acetat (**19**) gegenüber Nonivamid bei ähnlicher Gesamtintensität sowie die deutlich schnellere Abnahme der Schärfe.

### Beispiel 9: Kombination von Homovanillinsäureestern mit Paradieskörnerextrakt, Nonivamid und einem Capsicum-Extrakt

Es wurden verschiedene Kombinationen von Ethyl-2-(4-hydroxy-3-methoxy-phenyl)acetat (**17**) und Butyl-2-(4-hydroxy-3-methoxy-phenyl)acetat (**19**) mit Paradieskörnerextrakt (PN 300953, Symrise), Nonivamid und Capsicum-Extrakt 1.000.000 SHU sensorisch bewertet. Die Verkostungslösungen aus einem Homovanillinsäureester in ansteigender Konzentration (ppm) in Kombination mit einer trigeminalen Substanz bestimmter Konzentration auf Basis einer 5%igen Zuckerlösung (Tab. 3) wurden von 3 Prüfern bewertet. Hierfür wurde der Mundraum jeweils mit ca. 5 mL der jeweiligen Verkostungslösung gespült und die Lösung wieder ausgespuckt.

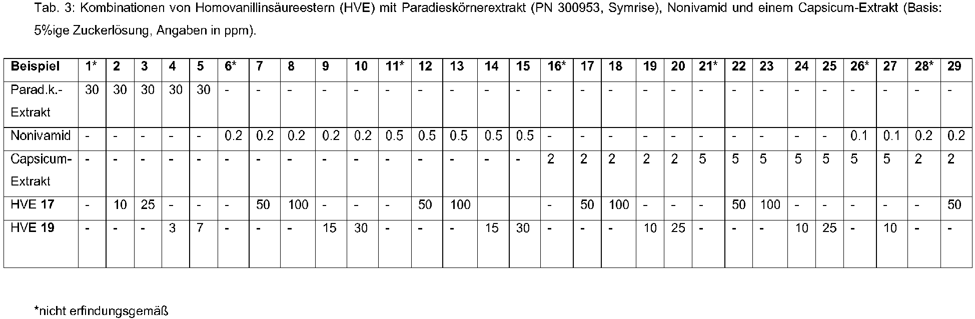

Homovanillinsäureester haben einen verstärkenden Einfluss auf die eingesetzten Verbindungen. Für die Kombination von Paradieskörnerextrakt (PN 300953, Symrise) mit Ethyl-2-(4-hydroxy-3-methoxy-phenyl)acetat (**17**, Beispiele 2 und 3) wurde ein schnelleres Einsetzen der Schärfe festgestellt. Bei der Kombination von Ethyl-2-(4-hydroxy-3-methoxy-phenyl)acetat (**17**) mit Nonivamid (Beispiele 7 und 8 sowie 12 und 13) wurde in beiden geprüften Konzentrationen die Schärfe von Nonivamid verstärkt, wobei diese Verstärkung mehr als dem additiven Effekt entsprach. Weiterhin wurde ein schnelleres Einsetzen der Schärfe festgestellt. Derselbe verstärkende und schneller einsetzende Effekt wurde auch in der Kombination von Ethyl-2-(4-hydroxy-3-methoxyphenyl)acetat (**17**) mit 2 ppm Capsicum-Extrakt 1.000.000 SHU festgestellt (Beispiele 17 und 18), wobei die Schärfe als angenehm und nicht lang nachklingend beurteilt wurde. Ohne die Kombination mit einem Homovanillylester wurde der Eintritt der Schärfe von Capsicum-Extrakt 1.000.000 SHU erst verzögert wahrgenommen (Beispiele 16* und 21*). Die Verstärkung der Schärfe war bei 2 ppm Capsicum-Extrakt 1.000.000 SHU deutlicher als bei 5 ppm. Bei der Kombination von 5 ppm Capsicum-Extrakt 1.000.000 SHU mit verschiedenen Mengen Ethyl-2-(4-hydroxy-3-methoxy-phenyl)acetat (**17**, Beispiele 22 und 23) wurde ein schnelles Abklingen der Schärfe deutlich. Zusätzlich wurde die Schärfe von Capsicum-Extrakt 1.000.000 SHU in der Kombination mit Ethyl-2-(4-hydroxy-3-methoxy-phenyl)acetat (**17**) in allen Konzentrationen um einen angenehmen wärmenden Effekt erweitert, der nach Abklingen des Schärfeempfindens zurückblieb. Für Butyl-2-(4-hydroxy-3-methoxy-phenyl)acetat (**19**) wurden dieselben Effekte bereits bei geringeren Konzentrationen festgestellt (Beispiele 19 und 20 sowie 24 und 25), wobei die Verstärkungen ebenfalls deutlicher als einem additiven Effekt waren. Auch eine Kombination von Nonivamid, Capsicum-Extrakt 1.000.000 SHU und Ethyl-2-(4-hydroxy-3-methoxy-phenyl)acetat (**17**, Beispiel 27) oder Butyl-2-(4-hydroxy-3-methoxyphenyl)acetat (**19**, Beispiel 29) zeigte zusätzlich zu den Kombinationen ohne Homovanillinsäureester (Beispiele 26* und 28*) ein schnelleres Abklingen der Schärfe sowie zusätzlich einen wärmenden Effekt.

### Beispiel 10: Verstärkung der Alkoholschärfe einer ethanolischen Lösung durch Ethyl-2-(4-hydroxy-3-methoxy-phenyl)acetat (17)

Auf der Basis einer 5%igen Zuckerlösung mit 20% Ethanol (Beispiel 1) wurden verschiedene Konzentrationen (10-100 ppm) von Ethyl-2-(4-hydroxy-3-methoxyphenyl)acetat (**17**), Flavon polymethoxyliert PMF 60 (Miritz) sowie Kombinationen (10-50 ppm) von Ethyl-2-(4-hydroxy-3-methoxy-phenyl)acetat (**17**) mit Flavon polymethoxyliert PMF 60 (Miritz) (20 ppm) mit einer zweiten Basislösung (5%ige Zuckerlösung mit 40% Ethanol) verglichen (Tab. 4). Hierfür wurde von 5 Prüfern der Mundraum jeweils mit ca. 5 mL der jeweiligen Verkostungslösung gespült, die Lösung wieder ausgespuckt und der Geschmackseindruck bewertet.

**Tab. 4: Kombinationen von Ethyl-2-(4-hydroxy-3-methoxy-phenyl)acetat (17) und Flavon polymethoxyliert PMF 60 (Miritz) in einer 5%igen Zuckerlösung mit 20% Ethanol (Angaben in ppm).**

| **Inhaltsstoff** | **1*** | **2** | **3** | **4** | **5** | **6*** | **7** | **8** | **9** |
|---|---|---|---|---|---|---|---|---|---|
| Ethyl-2-(4-hydroxy-3-methoxyphenyl)acetat (**17**) | - | 10 | 20 | 50 | 100 | - | 10 | 20 | 50 |
| Flavon polymethoxyliert PMF 60 | - | - | - | - | - | 20 | 20 | 20 | 20 |

| | | | | | | | | | |
|---|---|---|---|---|---|---|---|---|---|
| *nicht erfindungsgemäß | | | | | | | | | |

Die Basislösung (5%ige Zuckerlösung mit 20% Ethanol, Beispiel 1) wurde als alkoholisch, brennend beschrieben. Die Zugabe von Ethyl-2-(4-hydroxy-3-methoxyphenyl)acetat (**17**, Beispiel 2) führte zu einer Verstärkung und einer Verlängerung des brennenden Mundgefühls. Weitere Erhöhung auf 20 ppm und 50 ppm (Beispiele 2-5) ergab zusätzlich einen Schärfeeffekt. Flavon polymethoxyliert PMF 60 alleine (Beispiel 6) ergab in der Verkostungslösung eine Verstärkung des alkoholischen Geschmacks, aber kein brennendes Mundgefühl im Vergleich zu Beispiel 1. Durch die Kombination mit Ethyl-2-(4-hydroxy-3-methoxy-phenyl)acetat (**17**, Beispiele 7-9) wurde dieser Geschmack verstärkt, zusätzlich eine Verstärkung des brennenden Gefühls erzeugt und auch eine Verlängerung dieser Effekte erreicht, wobei der Einsatz von 20-50 ppm Ethyl-2-(4-hydroxy-3-methoxy-phenyl)acetat (**17**) sensorisch bevorzugt wurde. Im Vergleich zur zweiten Basislösung (5%igen Zuckerlösung mit 40% Ethanol) mit einem höheren Alkoholgehalt und starkem nasalen Effekt zeigte diese Kombination keine nasale Einwirkung.

### Beispiel 11: Wärmender Effekt von Homovanillinsäureestern im Vergleich zu Vanillylbutylether

Es wurden Testlösungen mit 4-10 ppm Homovanillinsäureester in einer 5%igen Zuckerlösung sensorisch bewertet und mit einer Testlösung von 10 ppm Vanillylbutylether verglichen. Ethyl-2-(4-hydroxy-3-methoxy-phenyl)acetat (**17**) hatte einen milderen Wärmeeffekt im Vergleich zu Vanillylbuthylether. Butyl-2-(4-hydroxy-3-methoxy-phenyl)acetat (**19**) hatte einen deutlicheren Wärmeeffekt als Vanillylbuthylether. Isobutyl-2-(4-hydroxy-3-methoxy-phenyl)acetat (**9**) zeigte einen Wärmeeffekt ähnlich zu Vanillylbuthylether, der aber länger anhielt. Propyl-2-(4-hydroxy-3-methoxy-phenyl)acetat (**18**) zeigte ebenfalls einen langanhaltenden Wärmeeffekt, der aber vergleichsweise später eintrat.

### Anwendungsbeispiele

### Anwendungsbeispiel 1: Typisch scharfe Aromakompositionen, enthaltend Homovanillinsäureester

Es wurden vermischt (alle Angaben, soweit nicht anders vermerkt, in Gew-%):

| **Inhaltsstoff** | **A** | **B** | **C** | **D** |
|---|---|---|---|---|
| 10 Gew.-% Pellitorin in 1,2-Propylenglycol/Diethylmalonat | 0.25 | 0.25 | 0.25 | 0.25 |
| Hesperetin | 2.50 | 2.50 | 2.50 | 2.50 |
| Phloretin | 1.50 | 1.50 | 1.50 | 1.50 |
| Ethyl-2-(4-hydroxy-3-methoxy-phenyl)acetat (**17**) | 3.00 | - | 1.50 | 1.00 |
| Butyl-2-(4-hydroxy-3-methoxy-phenyl)acetat (**19**) | - | 0.50 | 0.25 | - |
| Propyl-2-(4-hydroxy-3-methoxy-phenyl)acetat (**18**) | - | - | - | 1.50 |
| Propylenglycol | ad 100 | ad 100 | ad 100 | ad 100 |

Die Inhaltsstoffe (Stoffe bzw. Lösungen) werden in den oben angegebenen Mengenverhältnissen gemischt und dann mit Propylenglycol aufgenommen und durch leichtes Erwärmen vollständig gelöst.

### Anwendungsbeispiel 2: Sprühgetrocknete Aromakompositionen, enthaltend Homovanillinsäureester

Es wurden vermischt (alle Angaben, soweit nicht anders vermerkt, in Gew-%):

| **Inhaltsstoff** | **A** | **B** | **C** | **D** |
|---|---|---|---|---|
| Ethyl-2-(4-hydroxy-3-methoxy-phenyl)acetat (**17**) | 10.00 | - | - | - |
| Butyl-2-(4-hydroxy-3-methoxy-phenyl)acetat (**19**) | - | 3.50 | - | - |
| Propyl-2-(4-hydroxy-3-methoxy-phenyl)acetat (**18**) | - | - | 5.00 | - |
| Hexyl-2-(4-hydroxy-3-methoxy-phenyl)acetat (**21**) | - | - | - | 2.50 |
| Maltodextrin | ad 100 | ad 100 | ad 100 | ad 100 |

Die beiden Bestandteile werden in einer Mischung aus Ethanol und demineralisiertem Wasser gelöst und anschließend sprühgetrocknet.

### Anwendungsbeispiel 3: Herstellung von Mundwasser-Aromen unter Verwendung der oben beschriebenen Geschmackstoffe:

Es wurden vermischt (alle Angaben, soweit nicht anders vermerkt, in Gew-%):

| **Inhaltsstoff** | **A** | **B** | **C** | **D** | **E** |
|---|---|---|---|---|---|
| Anethol | 30 | 30 | 30 | 30 | 30 |
| Eucalyptol | 25 | 25 | 25 | 25 | 25 |
| L-Menthol | 36.00 | 36.00 | 38.00 | 36.20 | 36.00 |
| Optacool A | | 8 | | 8 | |
| Coolact 10 | 5 | | 5 | | 5 |
| Ethyl-2-(4-hydroxy-3-methoxyphenyl)acetat (**17**) | 4.00 | | | | 3.00 |
| Butyl-2-(4-hydroxy-3-methoxyphenyl)acetat (**19**) | | 1.00 | | | |
| P ropyl-2-(4-hydroxy-3-methoxyphenyl)acetat (**18**) | | | 2.00 | | 1.00 |
| Hexyl-2-(4-hydroxy-3-methoxyphenyl)acetat (**21**) | | | | 0.80 | |
| Total | 100 | 100 | 100 | 100 | 100 |

### Anwendungsbeispiel 4: Herstellung von Aromen mit Pfefferminzgeschmack unter Verwendung von erfindungsgemäßen Substanzen:

Es wurden vermischt (alle Angaben, soweit nicht anders vermerkt, in Gew-%):

| **Inhaltsstoff** | **A** | **B** | **C** | **D** | **E** | **F** |
|---|---|---|---|---|---|---|
| Pfefferminzöl *Mentha arvensis* | 56 | 59.00 | 58.50 | 58.80 | 56 | 59 |
| L-Menthon | 20 | 20 | 20 | 20 | 20 | 20 |
| L-Menthol | 20 | 20 | 20 | 20 | 20 | 20 |
| Ethyl-2-(4-hydroxy-3-methoxy-phenyl)acetat (**17**) | 4.00 | | | | 3.00 | |
| Butyl-2-(4-hydroxy-3-methoxy-phenyl)acetat (**19**) | | 1.00 | | | | 0.50 |
| Propyl-2-(4-hydroxy-3-methoxy-phenyl)acetat (**18**) | | | 1.50 | | 1.00 | 0.50 |
| Pentyl-2-(4-hydroxy-3-methoxy-phenyl)acetat (**20**) | | | | 1.20 | | |
| Total | 100 | 100 | 100 | 100 | 100 | 100 |

Die Aromakompositionen wurden in nachfolgend beschriebenen Anwendungsbeispielen eingesetzt.

### Anwendungsbeispiel 5: Alkoholreduziertes Getränk

Herstellung eines Biermix-Getränkes mit reduziertem Alkoholgehalt oder ohne Alkohol. Alle Angaben sind in Gew-%.

| **Bestandteil** | **A*** | **B** | **C** | **D** | **E** |
|---|---|---|---|---|---|
| Zuckersirup | 4 | 4 | 4 | 4 | 4 |
| Bier (4.9% vol.) | 50 | - | - | - | - |
| Bier (alkohol- und kalorienreduziert, 2.8% vol.) | - | - | 50 | - | 50 |
| Bier (alkoholfrei, 0%) | - | 50 | - | 50 | - |
| Zitronensäure | 0.15 | 0.15 | 0.15 | 0.15 | 0.15 |
| Ascorbinsäure | 0.01 | 0.01 | 0.01 | 0.01 | 0.01 |
| Grapefruitsaft | 6 | 6 | 6 | 6 | 6 |
| Grapefruitaroma | 0.20 | 0.20 | 0.20 | 0.20 | 0.20 |
| Aromakomposition (Anwendungsbeispiel 1A) | - | 0.20 | - | 0.20 | - |
| Aromakomposition (Anwendungsbeispiel 1B) | - | - | 0.20 | - | 0.20 |
| Wasser | ad 100 | ad 100 | ad 100 | ad 100 | ad 100 |
| Kohlensäure | 0.70 | 0.70 | 0.70 | 0.70 | 0.70 |

| | | | | | |
|---|---|---|---|---|---|
| *nicht erfindungsgemäß | | | | | |

### Anwendungsbeispiel 6: Anwendungen in einer Zahnpasta (,Silica opaque')

Alle Angaben, soweit nicht anders vermerkt, in Gew-%.

| **Bestandteil** | **A** | **B** | **C** | **D** | **E** | **F** |
|---|---|---|---|---|---|---|
| Entionisiertes Wasser | 26.53 | 26.53 | 26.53 | 26.53 | 26.53 | 26.53 |
| Sorbitol 70% | 45 | 44.975 | 45 | 44.975 | 45 | 44.975 |
| Solbrol M Na-Salz | 0.15 | 0.15 | 0.15 | 0.15 | 0.15 | 0.15 |
| Trinatriumphosphat | 0.10 | 0.10 | 0.10 | 0.10 | 0.10 | 0.10 |
| Saccharin | 0.20 | 0.20 | 0.20 | 0.20 | 0.20 | 0.20 |
| Natriummonofluorphosphat | 1.12 | 1.12 | 1.12 | 1.12 | 1.12 | 1.12 |
| PEG 1500 | 5 | 5 | 5 | 5 | 5 | 5 |
| Sident 9 (Abrasive Silica) | 10 | 10 | 10 | 10 | 10 | 10 |
| Sident 22 S (Dickende Silica) | 8 | 8 | 8 | 8 | 8 | 8 |
| Natriumcarboxymethylcellulose | 0.90 | 0.90 | 0.90 | 0.90 | 0.90 | 0.90 |
| Titan (IV) oxid | 0.50 | 0.50 | 0.50 | 0.50 | 0.50 | 0.50 |
| Natriumlaurylsulfat (SLS) | 1.50 | 1.50 | 1.50 | 1.50 | 1.50 | 1.50 |
| Pellitorin-Lösung PLM (enthaltend 10% Pellitorin) | - | 0.025 | - | 0.025 | - | 0.025 |
| Aroma Typ Pfefferminze (Anwendungsbeispiel 4A) | 1.00 | 1.00 | | | | |
| Aroma Typ Pfefferminze (Anwendungsbeispiel 4B) | | | 1.00 | 1.00 | | |
| Aroma Typ Pfefferminze (Anwendungsbeispiel 4C) | | | | | 1.00 | 1.00 |
| Total | 100 | 100 | 100 | 100 | 100 | 100 |

### Anwendungsbeispiel 7: Anwendung in einer Zahnpasta (Calciumcarbonat-Base)

Alle Angaben, soweit nicht anders vermerkt, in Gew-%.

| **Bestandteil** | **A** | **B** | **C** | **D** | **E** | **F** |
|---|---|---|---|---|---|---|
| Entionisiertes Wasser | 27.5 | 27.48 | 27.5 | 27.48 | 27.5 | 27.48 |
| Saccharin | 0.20 | 0.20 | 0.20 | 0.20 | 0.20 | 0.20 |
| Solbrol M Natriumsalz | 0.20 | 0.20 | 0.20 | 0.20 | 0.20 | 0.20 |
| Natriummonofluorphosphat | 0.80 | 0.80 | 0.80 | 0.80 | 0.80 | 0.80 |
| Sorbitol 70% | 29 | 29 | 29 | 29 | 29 | 29 |
| Calciumcarbonat | 35 | 35 | 35 | 35 | 35 | 35 |
| Sident 22 S (Verdickende Silica) | 2.50 | 2.50 | 2.50 | 2.50 | 2.50 | 2.50 |
| Natriumcarboxymethylcellulose | 1.30 | 1.30 | 1.30 | 1.30 | 1.30 | 1.30 |
| Titandioxid | 0.50 | 0.50 | 0.50 | 0.50 | 0.50 | 0.50 |
| Natriumlaurylsulfat | 2 | 2 | 2 | 2 | 2 | 2 |
| Pellitorin-Lösung PLM (enthaltend 10% Pellitorin) | - | 0.02 | - | 0.02 | - | 0.02 |
| Aroma Typ Pfefferminze (Anwendungsbeispiel 4D) | 1.00 | 1.00 | | | | |
| Aroma Typ Pfefferminze (Anwendungsbeispiel 4E) | | | 1.00 | 1.00 | | |
| Aroma Typ Pfefferminze (Anwendungsbeispiel 4F) | | | | | 1.00 | 1.00 |
| Total | 100 | 100 | 100 | 100 | 100 | 100 |

Durch die Verwendung der erfindungsgemäßen Substanzen wird ein schnell einsetzendes angenehmes Schärfeprofil mit wärmenden Aspekten erreicht. Zusätzlich wird ein verstärkter mundwässernder Effekt erzielt.

### Anwendungsbeispiel 8: Anwendung in einem zuckerfreien Kaugummi

| **Teil** | **Inhaltsstoff** | **Gew**.-**%** |
|---|---|---|
| A | Kaugummibase, Company "Jagum T" | 30.00 |
| B | Sorbit, pulverisiert | 39.00 |
| | Isomalt® (Palatinit GmbH) | 9.50 |
| | Xylit | 2.00 |
| | Mannit | 3.00 |
| | Aspartam® | 0.10 |
| | Acesulfam® K | 0.10 |
| | Emulgum® (Colloides Naturels, Inc.) | 0.30 |
| C | Sorbitol, 70 % | 14.00 |
| | Glycerin | 1.00 |
| D | Aromakompositionen gemäß Anwendungsbeispiel 4 | 1.00 |

Die Teile A bis D werden gemischt und intensiv geknetet. Die erhaltene Rohmasse kann dann z.B. in Form von dünnen Streifen zu verzehrfertigen Kaugummis verarbeitet werden.

### Anwendungsbeispiel 9: Mundwasser ("ready to use" ohne Alkohol)

Alle Angaben, soweit nicht anders vermerkt, in Gew-%.

| **Bestandteil** | **A** | **B** | **C** | **D** | **E** | **F** |
|---|---|---|---|---|---|---|
| Cremophor RH 455 | 1.80 | 1.80 | 1.80 | 1.80 | 1.80 | 1.80 |
| Entionisiertes Wasser | 87.57 | 87.5575 | 87.57 | 87.5575 | 87.57 | 87.5575 |
| Sorbitol 70% | 10 | 10 | 10 | 10 | 10 | 10 |
| Natriumfluorid | 0.18 | 0.18 | 0.18 | 0.18 | 0.18 | 0.18 |
| Natriumsaccharin 450 | 0.10 | 0.10 | 0.10 | 0.10 | 0.10 | 0.10 |
| Solbrol M Natriumsalz | 0.15 | 0.15 | 0.15 | 0.15 | 0.15 | 0.15 |
| Pellitorin-Lösung PLM (enthaltend 10% Pellitorin) | - | 0.0125 | - | 0.0125 | - | 0.0125 |
| Mundwasser Aroma (Anwendungsbeispiel 3A) | 0.20 | 0.20 | - | - | - | - |
| Mundwasser Aroma (Anwendungsbeispiel 3B) | - | - | 0.20 | 0.20 | - | - |
| Mundwasser Aroma (Anwendungsbeispiel 3C) | - | - | - | - | 0.20 | 0.20 |
| Total | 100 | 100 | 100 | 100 | 100 | 100 |

### Anwendungsbeispiel 10: Zahncreme und Mundwasser als 2-in-1 Produkt

Alle Angaben sind in Gew-%.

| **Bestandteil** | **A** | **B** | **C** | **D** |
|---|---|---|---|---|
| Ethanol, 96%ig | 5 | 5 | 5 | 5 |
| Sorbitol, 70 %ig in Wasser | 40 | 40 | 40 | 40 |
| Glycerin | 20 | 20 | 20 | 20 |
| Saccharin | 0.20 | 0.20 | 0.20 | 0.20 |
| Na-Monofluorphosphat | 0.76 | 0.76 | 0.76 | 0.76 |
| Solbrol M, Na-Salz | 0.15 | 0.15 | 0.15 | 0.15 |
| Abrasivkieselsäure (Sident 9) | 20 | 20 | 20 | 20 |
| Verdickungskieselsäure (Sident 22S) | 2 | 2 | 2 | 2 |
| Na-Carboxymethylcellu lose | 0.30 | 0.30 | 0.30 | 0.30 |
| Natriumlaurylsulfat | 1.20 | 1.20 | 1.20 | 1.20 |
| Grüner Farbstoff (1%ig in Wasser) | 0.50 | 0.50 | 0.50 | 0.50 |
| Wasser dest. | 9.39 | 9.39 | 9.39 | 9.39 |
| Aromakomposition (Anwendungsbeispiel 3A) | 0.50 | | | |
| Aromakomposition (Anwendungsbeispiel 3B) | | 0.50 | | |
| Aromakomposition (Anwendungsbeispiel 3C) | | | 0.50 | |
| Aromakomposition (Anwendungsbeispiel 3D) | | | | 0.50 |
| Total | 100 | 100 | 100 | 100 |

### Anwendungsbeispiel 11: Anwendung in Kombination mit einem scharfen Pflanzenextrakt als Alkoholverstärker

| Vergleichsprobe Likörbase 10%vol | |
|---|---|
| 7.39 kg | Alkohol, p.A.-Ware |
| 20 kg | Invertzuckersirup, 66.5 % Trockenmasse |
| 72.61 kg | Wasser |
| Summe 100 kg | |

| Likorbase 5.5%vol | |
|---|---|
| 4.06 kg | Alkohol, p.A.-Ware |
| 20 kg | Invertzuckersirup, 66.5 % Trockenmasse |
| 75.94 kg | Wasser |
| Summe 100 kg | |

Version A: Likörbase 5.5 %vol + 0.3 % einer 10 %igen Lösung eines Paradieskörnerextrakts in Ethanol
Version B: Likörbase 5.5 %vol + 0.075% einer 10 %igen Lösung eines Paradieskörnerextrakts in Ethanol + 0.2% einer Lösung von Ethyl-2-(4-hydroxy-3-methoxy-phenyl)acetat (17) 1 %ig in Ethanol (entspricht 20 ppm).

Bei Version B wird sensorisch die Alkoholscharfe der Vergleichsprobe besser imitiert als in Version A. Version A und die Vergleichsprobe sind sensorisch sehr ähnlich zu bewerten.

### Anwendungsbeispiel 12: Fußbalsam

| | **Gew.-%** | **Inhaltsstoff (INCI)** |
|---|---|---|
| A | 2.00 | Ceteareth-6, Stearylalkohol |
| | 2.00 | Ceteareth-25 |
| | 5.00 | Cetearylethylhexanoat |
| | 4.00 | Cetylalkohol |
| | 4.00 | Glycerylstearat |
| | 5.00 | Mineralöl |
| | 0.20 | Menthol |
| | 0.50 | Kampfer |
| B | 69.30 | Aqua dem. |
| | q.s. | Konservierungsmittel |
| C | 1.00 | Bisabolol |
| | 1.00 | Tocopherylacetat |
| D | 1.00 | wässrige Lösung mit 1-1.5 % Pentyl-2-(4-hydroxy-3-methoxyphenyl)acetat (**20**) |
| | 5.00 | Zaubernussextrakt |
| | 100 | Total |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Anwendungsbeispiel 13: Zuckerfreie Hartkaramelle

| **Inhaltsstoff** | **Gehalt [Gew**.**-%]** |
|---|---|
| Palatinit, Typ M | 75.47 |
| Wasser | 24.03 |
| Pfefferminzaroma | 0.10 |
| Aromazubereitung (Beispiel 1) | 0.40 |
| Total | 100 |

Zunächst wurde Palatinit mit Wasser gemischt. Die Mischung wurde dann bei 165 °C aufgeschmolzen und anschließend auf 115°C abgekühlt. Dann wurden das Pfefferminzaroma und die Aromazubereitung (Beispiel 1) zugegeben. Nach dem Durchmischen wurden die Mischungen in Formen gegossen, nach dem Erstarren aus den Formen entfernt und anschließend einzeln verpackt.

### Anwendungsbeispiel 14: Würzmischung, Typ "Pfeffer"

A = Vergleichszubereitung
B, C, D, E = erfindungsgemäße Zubereitungen

Alle Angaben sind in Gew-%.

| **Bestandteil** | **A*** | **B** | **C** | **D** | **E** |
|---|---|---|---|---|---|
| Milchprotein | 0.80 | 0.80 | 0.80 | 0.80 | 0.80 |
| Johannisbrotkernmehl | 2.00 | 2.00 | 2.00 | 2.00 | 2.00 |
| Maisstärke | 24.00 | 23.00 | 23.00 | 23.00 | 23.00 |
| Kochsalz | 14.00 | 14.00 | 14.00 | 14.00 | 14.00 |
| Paprikapulver | 12.00 | 12.00 | 12.00 | 12.00 | 12.00 |
| Tomatenpulver | 12.00 | 12.00 | 12.00 | 12.00 | 12.00 |
| Saccharose | 4.00 | 4.00 | 4.00 | 4.00 | 4.00 |
| Knoblauchpulver | 0.50 | 0.50 | 0.50 | 0.50 | 0.50 |
| Gehärtetes Pflanzenfett | 8.00 | 8.00 | 8.00 | 8.00 | 8.00 |
| Fettpulver | 11.00 | 11.00 | 11.00 | 11.00 | 11.00 |
| Natriumglutamat | 6.00 | 6.00 | 6.00 | 6.00 | 6.00 |
| Lebensmittelfarbstoff Rote Bete und Paprika | 2.00 | 2.00 | 2.00 | 2.00 | 2.00 |
| Aroma Typ "Pfeffer" | 2.00 | 2.00 | 2.00 | 2.00 | 2.00 |
| Aroma Typ "Pizza" | 1.20 | 1.20 | 1.20 | 1.20 | 1.20 |
| Aroma Typ "Tomate" | 0.40 | 0.40 | 0.40 | 0.40 | 0.40 |
| Extrakt aus schwarzem Pfeffer | 0.10 | 0.10 | 0.10 | 0.10 | 0.10 |
| Aromakomposition (Anwendungsbeispiel 1A) | - | 1.00 | - | - | - |
| Aromakomposition (Anwendungsbeispiel 1 B) | - | - | 1.00 | - | - |
| Aromakomposition (Anwendungsbeispiel 1C) | - | - | - | 1.00 | - |
| Aromakomposition (Anwendungsbeispiel 1D) | - | | | | 1.00 |
| Total | 100 | 100 | 100 | 100 | 100 |

| | | | | | |
|---|---|---|---|---|---|
| *nicht erfindungsgemäß | | | | | |

### Anwendungsbeispiel 15: Würzmischung für Kartoffelchips

A = Vergleichszubereitung
B, C, D, E = erfindungsgemäße Zubereitungen

Alle Angaben sind in Gew-%.

| **Bestandteil** | **A*** | **B** | **C** | **D** | **E** |
|---|---|---|---|---|---|
| Natriumglutamat | 3.50 | 3.50 | 3.50 | 3.50 | 3.50 |
| Käsepulver | 10.00 | 10.00 | 10.00 | 10.00 | 10.00 |
| Knoblauchpulver | 2.00 | 2.00 | 2.00 | 2.00 | 2.00 |
| Molkenpulver | 38.86 | 36.86 | 36.86 | 36.86 | 36.86 |
| Würzextraktöl | 0.20 | 0.20 | 0.20 | 0.20 | 0.20 |
| Paprikapulver | 9.80 | 9.80 | 9.80 | 9.80 | 9.80 |
| Kochsalz | 21.00 | 21.00 | 21.00 | 21.00 | 21.00 |
| Tomatenpulver | 9.00 | 9.00 | 9.00 | 9.00 | 9.00 |
| Trockenaroma | 2.50 | 2.50 | 2.50 | 2.50 | 2.50 |
| Siliciumdioxid | 0.02 | 0.02 | 0.02 | 0.02 | 0.02 |
| Pflanzenöl | 0.02 | 0.02 | 0.02 | 0.02 | 0.02 |
| Zwiebelpulver | 3.00 | 3.00 | 3.00 | 3.00 | 3.00 |
| Sahne Aromakonzentrat | 0.03 | 0.03 | 0.03 | 0.03 | 0.03 |
| Käse Aroma | 0.03 | 0.03 | 0.03 | 0.03 | 0.03 |
| Tomaten Aromakonzentrat | 0.04 | 0.04 | 0.04 | 0.04 | 0.04 |
| Sprühgetrocknete Zusammensetzung gemäß Anwendungsbeispiel 2A | - | 2.00 | - | - | - |
| Sprühgetrocknete Zusammensetzung gemäß Anwendungsbeispiel 2B | - | - | 2.00 | - | - |
| Sprühgetrocknete Zusammensetzung gemäß Anwendungsbeispiel 2C | - | - | - | 2.00 | - |
| Sprühgetrocknete Zusammensetzung gemäß Anwendungsbeispiel 2D | - | - | - | - | 2.00 |
| Total | 100 | 100 | 100 | 100 | 100 |

| | | | | | |
|---|---|---|---|---|---|
| *nicht erfindungsgemäß | | | | | |

6 g der Würzmischung wurden auf 94 g Kartoffelchips aufgezogen.

### Anwendungsbeispiel 16: Anwendung in einem Grünteegetränk

| **Inhaltsstoff** | **Einsatz in Gew.-%** |
|---|---|
| Grünteekonzentrat | 18.00 |
| 1% ige Lösung Ethyl-2-(4-hydroxy-3-methoxyphenyl)acetat (**17**) | 0.40 |
| demineralisiertes Wasser | 81.60 |
| Total | 100 |

Das Grünteekonzentrat wird mit der 1 %igen Lösung Ethyl-2-(4-hydroxy-3-methoxyphenyl)acetat (17) in Propylenglycol vermischt. Anschließend wird mit demineralisiertem Wasser aufgefüllt und erneut gründlich durchmischt. Dann wird das Produkt filtriert, verbrauchsfertig verpackt und bei 118°C sterilisiert. Der Geschmack wird durch ein Panel ausgebildeter Testpersonen als deutlich bevorzugt gegenüber dem nicht aromatisierten Grünteekonzentrat bewertet. Die Bitterkeit und Astringenz wird durch die Zugabe der erfindungsgemäßen Verbindungen reduziert.

### Anwendungsbeispiel 17: Verwendung in einem Eistee-Getränk (Schwarztee)

Die Homovanillinsäureester wurden jeweils 10 % in Ethanol vorgelöst. Schwarztee-Extrakt wurde in Wasser gelöst und zusammen mit Zucker, einer Aromazubereitung (Pfirsichgeschmack), sowie den ethanolischen Lösungen der Homovanillinsäureester in einem Becherglas verrührt.

| **Inhaltsstoff** | **Einsatz in Gew.-%** | |
|---|---|---|
| | **A** | **B** |
| Schwarztee-Extrakt | 1.40 | 1.40 |
| Wasser | 89.5 | 89.51 |
| Aromazubereitung (Typ Pfirsich) | 0.67 | 0.67 |
| Zucker | 7 | 7 |
| Citronensäure (kristallin) | 1.20 | 1.20 |
| Ascorbinsäure | 0.20 | 0.20 |
| 10 % in Ethanol Ethyl-2-(4-hydroxy-3-methoxy-phenyl)acetat **(17)** | 0.03 | - |
| 10 % in Ethanol Propyl-2-(4-hydroxy-3-methoxy-phenyl)acetat **(18)** | - | 0.02 |
| Total | 100 | 100 |

### Anwendungsbeispiel 18: Anwendung in einer Boullion

A = Vergleichszubereitung
B, C, D = erfindungsgemäße Zubereitungen

Alle Angaben sind in Gew-%.

| **Bestandteil** | **A*** | **B** | **C** | **D** |
|---|---|---|---|---|
| Fettpulver | 8.77 | 8.77 | 8.77 | 8.77 |
| Natriumglutamat | 8.77 | 8.77 | 8.77 | 8.77 |
| Hefeextrakt Pulver | 12.28 | 12.28 | 12.28 | 12.28 |
| Kochsalz | 29.83 | 29.83 | 29.83 | 29.83 |
| Maltodextrin | 37.28 | 36.68 | 37.13 | 36.98 |
| Natürlicher Gemüseextrakt | 3.07 | 3.07 | 3.07 | 3.07 |
| Ethyl-2-(4-hydroxy-3-methoxy-phenyl)acetat **(17)** | - | 0.60 | - | - |
| Butyl-2-(4-hydroxy-3-methoxy-phenyl)acetat **(19)** | - | - | 0.15 | - |
| Isobutyl-2-(4-hydroxy-3-methoxy-phenyl)acetat **(9)** | - | - | - | 0.30 |
| Total | 100 | 100 | 100 | 100 |

| | | | | |
|---|---|---|---|---|
| *nicht erfindungsgemäß | | | | |

15 g der jeweiligen Pulvermischung wurden mit je 1000 mL heißem Wasser aufgegossen.

### Anwendungsbeispiel 19: Instantsuppe, Typ Lauch-Creme

A = Vergleichszubereitung
B, C, D, E = erfindungsgemäße Zubereitungen

Alle Angaben sind in Gew-%.

| **Bestandteil** | **A*** | **B** | **C** | **D** | **E** |
|---|---|---|---|---|---|
| Kartoffelstärke | 20.00 | 20.00 | 20.00 | 20.00 | 20.00 |
| Fettpulver | 25.00 | 25.00 | 25.00 | 25.00 | 25.00 |
| Lactose | 20.00 | 20.00 | 20.00 | 20.00 | 20.00 |
| Maltodextrin | 11.73 | 11.65 | 11.68 | 11.69 | 11.66 |
| Kochsalz | 8.00 | 8.00 | 8.00 | 8.00 | 8.00 |
| Natriumglutamat | 3.00 | 3.00 | 3.00 | 3.00 | 3.00 |
| Spinatpulver | 2.00 | 2.00 | 2.00 | 2.00 | 2.00 |
| Grünes Lauchpulver | 2.00 | 2.00 | 2.00 | 2.00 | 2.00 |
| Citronensäure, als Pulver | 0.30 | 0.30 | 0.30 | 0.30 | 0.30 |
| Gehärtetes Pflanzenfett | 3.00 | 3.00 | 3.00 | 3.00 | 3.00 |
| Gefriergetrockneter Lauch | 1.00 | 1.00 | 1.00 | 1.00 | 1.00 |
| Huhnaroma | 1.00 | 1.00 | 1.00 | 1.00 | 1.00 |
| Würzmischung Typ "grüner Lauch", Pulver | 2.00 | 2.00 | 2.00 | 2.00 | 2.00 |
| Würzmischung, Typ "gekochte Zwiebel" | 0.60 | 0.60 | 0.60 | 0.60 | 0.60 |
| Hefe-Würzmischung, Typ "Gemüsebrühe", Pulver | 0.30 | 0.30 | 0.30 | 0.30 | 0.30 |
| Curcuma-Extrakt | 0.07 | 0.07 | 0.07 | 0.07 | 0.07 |
| Ethyl-2-(4-hydroxy-3-methoxy-phenyl)acetat **(17)** | - | 0.08 | - | - | 0.06 |
| Propyl-2-(4-hydroxy-3-methoxy-phenyl)acetat **(18)** | - | - | 0.05 | - | - |
| Isobutyl-2-(4-hydroxy-3-methoxy-phenyl)acetat **(8)** | - | - | - | 0.04 | 0.01 |
| Total | 100 | 100 | 100 | 100 | 100 |

5 g der jeweiligen Pulvermischung wurden mit je 100 mL heißem Wasser aufgegossen, um eine verzehrfertige Suppe zu erhalten.

### Anwendungsbeispiel 20: Instantsuppe, Typ Hühnersuppe mit Nudeln

A = Vergleichszubereitung
B, C, D, E = erfindungsgemäße Zubereitungen

Alle Angaben sind in Gew-%.

| **Bestandteil** | **A*** | **B** | **C** | **D** | **E** |
|---|---|---|---|---|---|
| Stärke | 16.16 | 16.06 | 16.12 | 16.13 | 16.11 |
| Kochsalz | 7.00 | 7.00 | 7.00 | 7.00 | 7.00 |
| Saccharose, raffiniert | 3.20 | 3.20 | 3.20 | 3.20 | 3.20 |
| Natriumglutamat | 3.20 | 3.20 | 3.20 | 3.20 | 3.20 |
| Natriuminosinat / Natriumguanylat im Verhältnis 1:1 | 0.80 | 0.80 | 0.80 | 0.80 | 0.80 |
| Säurehydrolysiertes Pflanzenprotein | 8.00 | 8.00 | 8.00 | 8.00 | 8.00 |
| Fettpulver | 2.00 | 2.00 | 2.00 | 2.00 | 2.00 |
| Gemüsefett, sprühgetrocknet | 1.00 | 1.00 | 1.00 | 1.00 | 1.00 |
| Gefriergetrocknetes Hühnerfleisch, in Stückchen | 2.00 | 2.00 | 2.00 | 2.00 | 2.00 |
| Suppen-Nudeln | 32.00 | 32.00 | 32.00 | 32.00 | 32.00 |
| Maltodextrin | 12.00 | 12.00 | 12.00 | 12.00 | 12.00 |
| Chinesisches Gemüse, gefriergetrocknet | 4.60 | 4.60 | 4.60 | 4.60 | 4.60 |
| Huhnaroma | 8.00 | 8.00 | 8.00 | 8.00 | 8.00 |
| Lebensmittelfarbstoff Riboflavin | 0.04 | 0.04 | 0.04 | 0.04 | 0.04 |
| Ethyl-2-(4-hydroxy-3-methoxy-phenyl)acetat **(17)** | - | 0.10 | - | - | 0.04 |
| Butyl-2-(4-hydroxy-3-methoxy-phenyl)acetat **(19)** | - | - | 0.04 | - | 0.01 |
| Hexyl-2-(4-hydroxy-3-methoxy-phenyl)acetat **(21)** | - | - | - | 0.03 | - |
| Total | 100 | 100 | 100 | 100 | 100 |

| | | | | | |
|---|---|---|---|---|---|
| *nicht erfindungsgemäß | | | | | |

4.60 g der jeweiligen Pulvermischung wurden 10 Minuten in je 100 mL Wasser gekocht, um eine verzehrfertige Suppe zu erhalten.

### Anwendungsbeispiel 21: Herstellung von dunklen Chilli-Schokoladen unter Verwendung der erfindungsgemäßen Substanzen.

Alle Angaben, soweit nicht anders vermerkt, in Gew-%.
A = Vergleichszubereitung dunkle Schokolade
B = kalorienreduzierte dunkle Schokolade
C = kalorienreduzierte dunkle Schokolade
D = kalorienreduzierte dunkle Schokolade
E = kalorienreduzierte Vollmilchschokolade

| **Bestandteil** | **A*** | **B** | **C** | **D** | **E** |
|---|---|---|---|---|---|
| Kakaobutter | 14.49 | 12.99 | 13.49 | 9.48 | 14.00 |
| Cacaomasse | 41.00 | 39.00 | 42.00 | 44.00 | 23.00 |
| Erythritol | - | 47.45 | - | - | - |
| Maltitol, kristallin | - | - | - | 23.00 | |
| Inulin | - | - | - | 23.00 | |
| Sorbitol | - | - | 44.00 | - | - |
| Lactitol | - | - | - | - | 38.55 |
| Polydextrose | - | - | - | - | 9.70 |
| Vollmilchpulver | - | - | - | - | 14.00 |
| Sucrose | 43.98 | - | - | - | - |
| Lecitin | 0.48 | 0.48 | 0.40 | 0.48 | 0.50 |
| Vanillin | 0.02 | 0.02 | 0.02 | 0.02 | 0.20 |
| Aspartam | - | 0.03 | 0.06 | - | 0.03 |
| Capsicum-Extrakt (1.000.000 SHU) | 0.03 | 0.02 | 0.02 | 0.01 | - |
| Hexyl-2-(4-hydroxy-3-methoxy-phenyl)acetat **(21)** | - | 0.01 | - | 0.01 | 0.01 |
| Butyl-2-(4-hydroxy-3-methoxy-phenyl)acetat (**19**) | - | - | 0.01 | - | 0.01 |
| Total | 100 | 100 | 100 | 100 | 100 |

| | | | | | |
|---|---|---|---|---|---|
| *nicht erfindungsgemäß | | | | | |

Die in den vorstehenden Anwendungsbeispielen gefundenen Effekte lassen sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen und Mischungen aufgefasst werden muss. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile ist in der oben stehenden Beschreibung offenbart.

## Patentansprüche

1. Verwendung einer Verbindung der Formel (I) 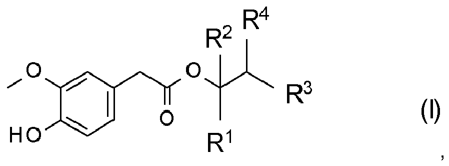 wobei
(i) R¹ und R² unabhängig voneinander ein Wasserstoffatom oder einen Alkylrest mit 1-2 Kohlenstoffatomen darstellen,
R³ und R⁴ unabhängig voneinander ein Wasserstoffatom oder einen linearen oder verzweigten Alkylrest mit 1 bis 5 Kohlenstoffatomen, einen Phenylrest, einen Alkylphenylrest oder einen linearen oder verzweigten Alkenylrest mit 2 bis 4 Kohlenstoffatomen oder einen Alkenylphenylrest darstellen,
oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
R² ein Wasserstoffatom oder einen Alkylrest mit 1-2 Kohlenstoffatomen darstellt,
R⁴ ein Wasserstoffatom oder einen linearen oder verzweigten Alkylrest mit 1 bis 5 Kohlenstoffatomen, einen Phenylrest, einen Alkylphenylrest oder einen linearen oder verzweigten Alkenylrest mit 2 bis 4 Kohlenstoffatomen oder einen Alkenylphenylrest darstellt,
oder eines physiologisch akzeptablen Salzes davon, wobei die phenolische Hydroxygruppe in Formel (I) deprotoniert ist, oder einer Mischung umfassend eine oder mehrere unterschiedliche Verbindungen der Formel (I) und/oder physiologisch akzeptable Salze davon, wobei jeweils die phenolische Hydroxygruppe in Formel (I) deprotoniert ist, oder bestehend aus mehreren unterschiedlichen Verbindungen der Formel (I) und/oder Salzen davon, wobei jeweils die phenolische Hydroxygruppe in Formel (I) deprotoniert ist,
als Aromastoff.

2. Verwendung nach Anspruch 1
- als Aromastoff bzw. Scharfstoff mit einem wärme- und/oder schärfeerzeugenden Effekt,
und/oder
- als Aromastoff zum Vermindern oder Maskieren eines unangenehmen Geschmackseindrucks, vorzugsweise ausgewählt aus der Gruppe bestehend aus adstringierend, bitter, trocken, staubig, mehlig, kalkig und metallisch,
und/oder
- als Aromastoff zum Verstärken eines angenehmen Geschmackseindrucks, vorzugsweise ausgewählt aus der Gruppe bestehend aus wärmend, scharf und kühlend.

3. Verwendung nach Anspruch 1 oder 2, wobei für die Verbindung der Formel (I) bzw. eine, mehrere oder sämtliche Verbindungen der Formel (I) unabhängig voneinander in der Mischung gilt:
(i) R¹ und R² stellen unabhängig voneinander ein Wasserstoffatom oder Methyl dar,
R³ und R⁴ stellen unabhängig voneinander ein Wasserstoffatom oder einen linearen oder verzweigten Alkylrest mit 1 bis 5 Kohlenstoffatomen dar,
oder
(ii) Formel (I) entspricht der folgenden Formel (la) 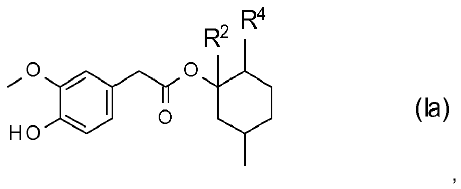
R² stellt ein Wasserstoffatom dar,
R⁴ stellt 2-Propyl dar.

4. Verwendung nach einem der vorangehenden Ansprüche, wobei für die Verbindung der Formel (I) bzw. eine, mehrere oder sämtliche Verbindungen der Formel (I) unabhängig voneinander in der Mischung gilt:
R¹ und R² stellen jeweils ein Wasserstoffatom dar,
R³ stellt ein Wasserstoffatom oder einen linearen oder verzweigten Alkylrest mit 1 bis 4 Kohlenstoffatomen dar,
R⁴ stellt ein Wasserstoffatom dar.

5. Verwendung nach einem der vorangehenden Ansprüche, wobei die Verbindung der Formel (I) bzw. eine, mehrere oder sämtliche Verbindungen der Formel (I) in der Mischung ausgewählt ist bzw. sind aus der Gruppe bestehend aus
2-Phenylethyl-2-(4-hydroxy-3-methoxy-phenyl)acetat **(1)** 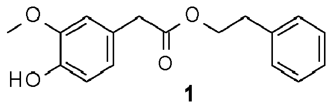
[(*E*)-Cinnamyl]-2-(4-hydroxy-3-methoxy-phenyl)acetat **(2)** 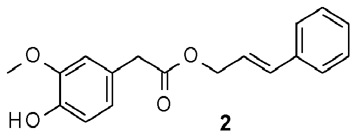
1-Ethylbutyl-2-(4-hydroxy-3-methoxy-phenyl)acetat **(3)** 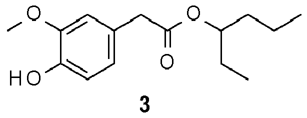
3-Methylbut-2-enyl-2-(4-hydroxy-3-methoxy-phenyl)acetat **(4)** 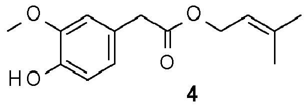
[(*E*)-Hex-2-enyl]-2-(4-hydroxy-3-methoxy-phenyl)acetat **(5)** 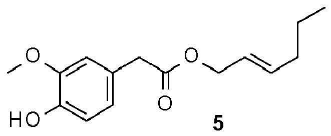
[(*Z*)-Hex-3-enyl]-2-(4-hydroxy-3-methoxy-phenyl)acetat **(6)** 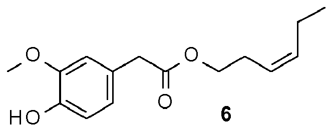
Isopropyl-2-(4-hydroxy-3-methoxy-phenyl)acetat **(7)** 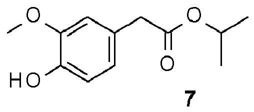
*sec*-Butyl-2-(4-Hydroxy-3-methoxy-phenyl)acetat **(8)** 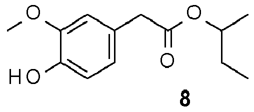
Isobutyl-2-(4-hydroxy-3-methoxy-phenyl)acetat **(9)** 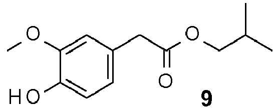
1,1-Dimethylpropyl 2-(4-hydroxy-3-methoxy-phenyl)acetat **(10)** 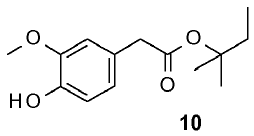
Isopentyl-2-(4-hydroxy-3-methoxy-phenyl)acetat **(11)** 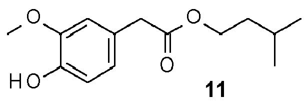
2-Methylbutyl-2-(4-hydroxy-3-methoxy-phenyl)acetat **(12)** 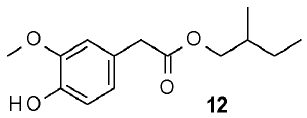
1-Methylpentyl-2-(4-hydroxy-3-methoxy-phenyl)acetat **(13)** 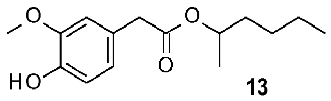
Heptyl-2-(4-hydroxy-3-methoxy-phenyl)acetat **(14)** 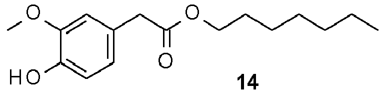
1-Methylhexyl-2-(4-hydroxy-3-methoxy-phenyl)acetat **(15)** 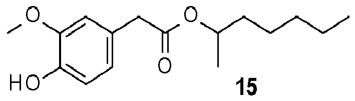
(2-Isopropyl-5-methyl-cyclohexyl)-2-(4-hydroxy-3-methoxy-phenyl)acetat **(16)** 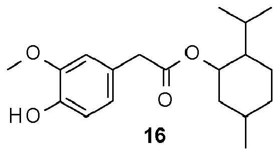
Ethyl-2-(4-hydroxy-3-methoxy-phenyl)acetat **(17)** 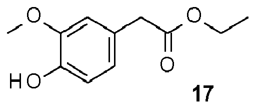
Propyl-2-(4-hydroxy-3-methoxy-phenyl)acetat **(18)** 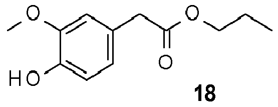
Butyl-2-(4-hydroxy-3-methoxy-phenyl)acetat **(19)** 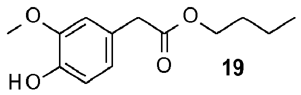
Pentyl-2-(4-hydroxy-3-methoxy-phenyl)acetat **(20)** 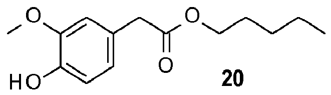
Hexyl-2-(4-hydroxy-3-methoxy-phenyl)acetat **(21)**

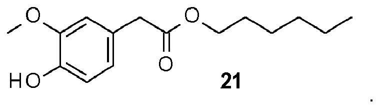

6. Verwendung nach einem der vorangehenden Ansprüche in einer pharmazeutischen, einer der Ernährung, der Mundhygiene oder dem Genuss dienenden Zubereitung, vorzugsweise wobei die Gesamtmenge an Verbindung(en) der Formel (I) und/oder Salz(en) davon in der Zubereitung ausreicht, um
(a) bei Gebrauch oder Verzehr der Zubereitung sensorisch einen wärmenden und/oder scharfen Effekt auf der Zunge oder im Mundraum hervorzurufen, und/oder
(b) einen unangenehmen Geschmackseindruck, vorzugsweise ausgewählt aus der Gruppe bestehend aus adstringierend, bitter, trocken, staubig, mehlig, kalkig und metallisch, zu vermindern oder zu maskieren,
und/oder
(c) einen angenehmen Geschmackseindruck, vorzugsweise ausgewählt aus der Gruppe bestehend aus wärmend, scharf und kühlend, zu verstärken.

7. Verwendung nach Anspruch 6, wobei die Gesamtmenge an Verbindung(en) der Formel (I) und/oder Salz(en) davon in der Zubereitung nicht ausreicht, um einen wärmenden oder scharfen Effekt auf der Zunge oder im Mundraum hervorzurufen, aber ausreicht, um einen unangenehmen Geschmackseindruck eines unangenehm schmeckenden Stoffes oder Stoffgemisches zu maskieren oder zu vermindern.

8. Verbindung der Formel (I) oder physiologisch akzeptables Salz davon, wobei die phenolische Hydroxygruppe in Formel (I) deprotoniert ist, oder Mischung umfassend eine oder mehrere unterschiedliche Verbindungen der Formel (I) und/oder ein oder mehrere physiologisch akzeptable Salze davon, wobei die phenolische Hydroxygruppe in Formel (I) jeweils deprotoniert ist, oder bestehend aus mehreren unterschiedlichen Verbindungen der Formel (I) und/oder physiologisch akzeptablen Salzen davon, wobei die phenolische Hydroxygruppe in Formel (I) jeweils deprotoniert ist, 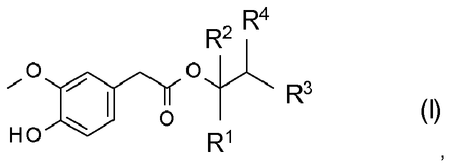 wobei
(i) R¹ und R² unabhängig voneinander ein Wasserstoffatom oder einen Alkylrest mit 1-2 Kohlenstoffatomen darstellen,
R³ und R⁴ unabhängig voneinander ein Wasserstoffatom oder einen linearen oder verzweigten Alkylrest mit 1 bis 5 Kohlenstoffatomen, einen Phenylrest, einen Alkylphenylrest oder einen linearen oder verzweigten Alkenylrest mit 2 bis 4 Kohlenstoffatomen oder einen Alkenylphenylrest darstellen,
oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
R² ein Wasserstoffatom oder einen Alkylrest mit 1-2 Kohlenstoffatomen darstellt,
R⁴ ein Wasserstoffatom oder einen linearen oder verzweigten Alkylrest mit 1 bis 5 Kohlenstoffatomen, einen Phenylrest, einen Alkylphenylrest oder einen linearen oder verzweigten Alkenylrest mit 2 bis 4 Kohlenstoffatomen oder einen Alkenylphenylrest darstellt,
mit der Maßgabe, dass
- R¹, R², R³ und R⁴ nicht sämtlich Wasserstoffatome darstellen,
und
- für den Fall, dass R¹, R² und R⁴ Wasserstoff darstellen, R³ weder einen linearen Alkylrest mit 1, 2, 4 oder 5 Kohlenstoffatomen, noch 2-Propyl, Phenyl oder Ethylphenyl darstellt,
und
- für den Fall, dass R², R³ und R⁴ Wasserstoff darstellen, R¹ nicht einen linearen Alkylrest mit 1 oder 2 Kohlenstoffatomen darstellt,
- nicht R³ und R⁴ Methyl darstellen, wenn R¹ und R² Wasserstoff darstellen, vorzugsweise weder R³, noch R⁴ Methyl darstellen, wenn R¹ und R² Wasserstoff darstellen,
und
- nicht R¹ und R² Methyl darstellen, wenn R³ und R⁴ Wasserstoff darstellen, vorzugsweise weder R1, noch R2 Methyl darstellen, wenn R³ und R⁴ Wasserstoff darstellen.

9. Verbindung oder Mischung nach Anspruch 8, wobei die Verbindung de Formel (I) bzw. eine, mehrere oder sämtliche Verbindungen der Formel (I) in der Mischung ausgewählt ist bzw. sind aus der Gruppe bestehend aus
[(*E*)-Cinnamyl]-2-(4-hydroxy-3-methoxy-phenyl)acetat **(2)** 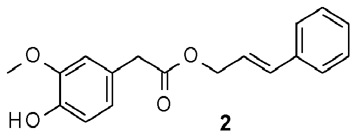
1-Ethylbutyl-2-(4-hydroxy-3-methoxy-phenyl)acetat **(3)** 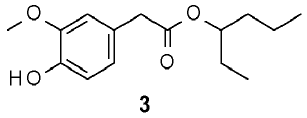
3-Methylbut-2-enyl-2-(4-hydroxy-3-methoxy-phenyl)acetat **(4)** 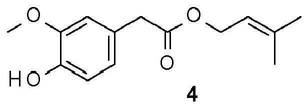
[(*E*)-Hex-2-enyl]-2-(4-hydroxy-3-methoxy-phenyl)acetat **(5)** 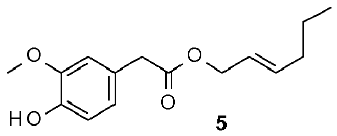
[(*Z*)-Hex-3-enyl]-2-(4-hydroxy-3-methoxy-phenyl)acetat **(6)** 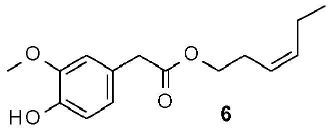
1,1-Dimethylpropyl 2-(4-hydroxy-3-methoxy-phenyl)acetat **(10)** 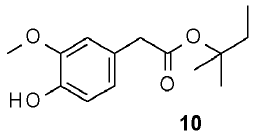
2-Methylbutyl-2-(4-hydroxy-3-methoxy-phenyl)acetat **(12)** 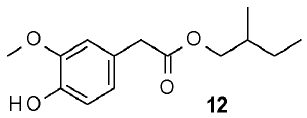
1-Methylpentyl-2-(4-hydroxy-3-methoxy-phenyl)acetat **(13)** 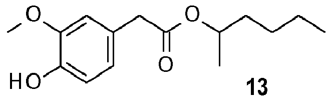
1-Methylhexyl-2-(4-hydroxy-3-methoxy-phenyl)acetat **(15)** 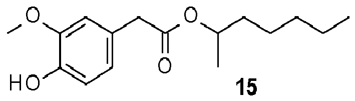
(2-Isopropyl-5-methyl-cyclohexyl)-2-(4-hydroxy-3-methoxy-phenyl)acetat **(16)** 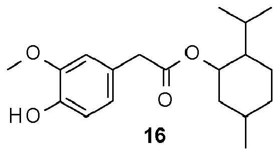
Pentyl-2-(4-hydroxy-3-methoxy-phenyl)acetat **(20)**

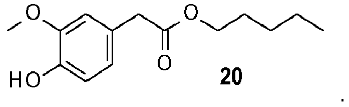

10. Aromakomposition
(A) umfassend oder bestehend aus einer Mischung nach Anspruch 8 oder 9, vorzugsweise wobei
die Gesamtmenge an Verbindung(en) der Formel (I) und/oder Salz(en) davon in der Aromakomposition, bezogen auf das Gesamtgewicht der Aromakomposition, im Bereich von 500 bis 100.000 mg/kg liegt, vorzugsweise im Bereich von 1.000 bis 40.000 mg/kg, besonders bevorzugt im Bereich von 1.000 bis 15.000 mg/kg
oder
(B) umfassend eine Verbindung der Formel (I), wie in einem der Ansprüche 1, 3, 4 oder 5 definiert, oder ein physiologisch akzeptables Salz davon, wie in einem der Ansprüche 1, 3, 4 oder 5 definiert, oder umfassend oder bestehend aus einer Mischung wie in einem der Ansprüche 1, 3, 4 oder 5 definiert,
wobei
die Gesamtmenge an Verbindung(en) der Formel (I) und/oder Salz(en) davon in der Aromakomposition, bezogen auf das Gesamtgewicht der Aromakomposition, im Bereich von 500 bis 100.000 mg/kg liegt, vorzugsweise im Bereich von 1.000 bis 40.000 mg/kg, besonders bevorzugt im Bereich von 1.000 bis 15.000 mg/kg.

11. Aromakomposition nach Anspruch 10, zudem umfassend einen oder mehrere weitere, nicht der Formel (I) entsprechende Aromastoffe, vorzugsweise ausgewählt aus der Gruppe bestehend aus
a) Wärme verursachende oder Scharfstoffe, bevorzugt ausgewählt aus der Liste bestehend aus: Capsaicinoiden, wie zum Beispiel Capsaicin, Dihydrocapsaicin oder Nonivamid; Gingerolen, wie zum Beispiel Gingerol-6, Gingerol-8, oder Gingerol-10; Shogaolen wie Shogaol-6, Shogaol-8, Shogaol-10; Gingerdionen, wie zum Beispiel Gingerdion-6, Gingerdion-8 oder Gingerdion-10; Paradolen, wie zum Beispiel Paradol-6, Paradol-8 oder Paradol-10; Dehydrogingerdionen, wie zum Beispiel Dehydrogingerdion-6, Dehydrogingerdion-8 oder Dehydrogingerdion-10; Piperin und Piperinderivaten;
b) als stechend oder beißend wahrnehmbare Stoffe, bevorzugt ausgewählt aus der Gruppe bestehend aus: aromatischen Isothiocyanaten, wie zum Beispiel Phenylethylisothiocyanat, Allylisothiocyanat, Cyclopropylisothiocyanat, Butylisothiocyanat, 3-Methylthiopropylisothiocyanat, 4-Hydroxybenzylisothiocyanat, 4-Methoxybenzylisothiocyanat;
c) als kribbelnd (tingling) beschriebenen Alkamide, vorzugsweise ausgewählt aus der Gruppe bestehend aus 2E,4E-Decadiensäure-N-isobutylamid (trans-Pellitorin) (insbesondere solche wie beschrieben in WO 2004/043906, die bezüglich der darin offenbarten entsprechenden Verbindungen auf dem Wege der Verweisung Bestandteil dieser Anmeldung wird); 2E,4Z-Decadiensäure-N-isobutylamid (cis-Pellitorin) (insbesondere solche wie beschrieben in WO 2004/000787, die bezüglich der darin offenbarten entsprechenden Verbindungen auf dem Wege der Verweisung Bestandteil dieser Anmeldung wird); 2Z,4Z- Decadiensäure-N-isobutylamid; 2Z,4E-Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy-2-methylpropyl)amid (gamma-Hydroxysanshool); 2E,4E,8Z, 10E, 12E-Tetradecapentaensäure-N-(2-hydroxy-2-methylpropyl)amid (gamma-Hydroxysanshool); 2E,4E,8E, 10E, 12E-Tetradecapentaensäure-N-(2-hydroxy-2-methylpropyl)amid (gamma-Hydroxyisosanshool); 2E,4E,8Z,10E,12E-Tetradecapentaensäure-N-(2-methyl-2-propenyl)amid (gamma-Dehydrosanshool); 2E,4E,8Z,10E,12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
d) Stoffe mit physiologischer Kühlwirkung, vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (I-Menthoxy)-1,2-propandiol, (I-Menthoxy)-2-methyl-1,2-propandiol, I-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), andere als in der vorliegenden Erfindung genannte Menthancarbonsäureamide (z.B. Menthancarbonsäure-N-ethylamid [WS3], Menthancarbonsäure-N-(p-methoxyphenyl)amid [SC1], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben), weitere Kühlwirkstoffe wie in WO2011061330 beschrieben, insbesondere Derivate verschieden substituierter Zimt- und 2-Phenoxysäuren, besonders bevorzugt Methylendioxyzimtsäure-N,N-diphenylamid, Methylendioxyzimtsäure-N-ethyl-N-phenylamid, Methylendioxyzimt-säure-N-pyridyl-N-phenylamid;
e) Stoffe mit adstringierender Wirkung, vorzugsweise ausgewählt aus der folgenden Liste: Catechine wie z.B. Epicatechine, Gallocatechine, Epigallocatechine sowie deren jeweiligen Gallussäureester, z.B. Epigallocatechingallat oder Epicatechingallat, dern Oligomere (Procyanidine, Proanthocyanidine, Prodelphinidine, Procyanirine, Thearubigenine, Theogalline) sowie deren C- und O-Glycoside; Dihydroflavonoide wie Dihydromyricetin, Taxifolin, sowie deren C- und O-Glycoside, Flavonole wie Myricetin, Quercetin sowie deren C- und O-Glycoside wie Quercetrin, Rutin, Gallussäaureester von Kohlenhydraten wie Tannin, Pentagalloylglucose oder deren Reaktionsprodukte wie Elligatannin, Aluminiumsalze, z.B. Alaun.

12. Aromakomposition nach Anspruch 10 oder 11, zudem umfassend einen oder mehrere nicht der Formel (I) entsprechende Stoffe mit unangenehmer, insbesondere bitterer Geschmacksqualität, oder einer adstringierenden, bitteren, trockenen, staubigen, mehligen, kalkigen und/oder metallischen Note, vorzugsweise ausgewählt aus der Gruppe bestehend aus:
f) Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallus- oder Elagsäureester von Kohlenhydraten, z.B. Pentagalloylglucose, Tanninsäuren), nichthydrolisierbare Tannine (ggfs. galloylierte Catechine, Gallocatechine, Epigallocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. gamma-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside);
g) Stoffe mit einem nicht unangenehmen Primärgeschmack (beispielsweise süß, salzig, würzig, sauer) und/oder -geruch, vorzugsweise ausgewählt aus der Gruppe der Süßstoffe oder Zuckeraustauschstoffe, vorzugsweise Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Rebaudiosid A, Rebaudiosid B, Rebaudiosid C, Rebaudiosid D, Rebaudiosid X, Rubusoside, Hernandulcin, Thaumatin, Miraculin, Glycyrrhizin, Glycyrrhetinsäure, Balansin A oder Balansin B, oder deren Derivate, Cyclamat oder die pharmazeutisch akzeptablen Salze der vorgenannten Verbindungen.

13. Pharmazeutische Zubereitung, der Ernährung, der Mundhygiene oder dem Genuss dienende Zubereitung, umfassend
(A) eine Mischung nach Anspruch 8 oder 9,
vorzugsweise wobei
die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
oder
(B) eine Verbindung der Formel (I), wie in einem der Ansprüche 1, 3, 4 oder 5 definiert, oder ein physiologisch akzeptables Salz davon, wie in einem der Ansprüche 1, 3, 4 oder 5 definiert, oder eine Mischung wie in einem der Ansprüche 1, 3, 4 oder 5 definiert,
wobei
die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
oder
(C) eine Aromakomposition nach Anspruch 10, 11 oder 12,
vorzugsweise wobei
die Gesamtmenge an Verbindung(en) der Formel (I) und/oder Salz(en) davon in der Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg.

14. Zubereitung nach Anspruch 13, zudem umfassend
ein oder mehrere übliche Grund-, Hilfs- und Zusatzstoffe in einer Menge von, bezogen auf das Gesamtgewicht der Zubereitung, 5 bis 99,999999 Gew.-%, vorzugsweise 10 bis 80 Gew.-%,
und/oder
Wasser in einer Menge, bezogen auf das Gesamtgewicht der Zubereitung, bis zu 99,999999 Gew.-%, vorzugsweise in einer Menge von 5 bis 80 Gew.-%.

15. Zubereitung nach Anspruch 13 oder 14, wobei die Gesamtmenge an Verbindung(en) der Formel (I) und/oder Salz(en) davon in der Zubereitung ausreicht, um
(a) bei Gebrauch oder Verzehr der Zubereitung sensorisch einen wärmenden und/oder scharfen Effekt auf der Zunge oder im Mundraum hervorzurufen, und/oder
(b) einen unangenehmen Geschmackseindruck, vorzugsweise eines anderen in der Zubereitung enthaltenen Stoffes, insbesondere einen Geschmackseindruck ausgewählt aus der Gruppe bestehend aus adstringierend, bitter, trocken, staubig, mehlig, kalkig und metallisch, zu vermindern oder zu maskieren,
und/oder
(c) einen angenehmen Geschmackseindruck, vorzugsweise eines anderen in der Zubereitung enthaltenen Stoffes, insbesondere eines Geschmackseindrucks ausgewählt aus der Gruppe bestehend aus wärmend, scharf und kühlend, zu verstärken.

16. Verfahren zum Herstellen einer pharmazeutischen Zubereitung, einer der Ernährung, der Mundhygiene oder dem Genuss dienenden Zubereitung, vorzugsweise einer Zubereitung nach einem der Ansprüche 13 bis 15, umfassend folgende Schritte:
i) Bereitstellen
(A) einer Mischung nach Anspruch 8 oder 9,
vorzugsweise wobei
die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
oder
(B) einer Verbindung der Formel (I), wie in einem der Ansprüche 1, 3, 4 oder 5 definiert, oder eines physiologisch akzeptablen Salz davon, wie in einem der Ansprüche 1, 3, 4 oder 5 definiert, oder einer Mischung wie in einem der Ansprüche 1, 3, 4 oder 5 definiert,
wobei
die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
oder
(C) eine Aromakomposition nach Anspruch 10, 11 oder 12, vorzugsweise wobei
die Gesamtmenge an Verbindung(en) der Formel (I) und/oder Salz(en) so ausgewählt ist, dass die Gesamtmenge in der herzustellenden Zubereitung, bezogen auf das Gesamtgewicht der Zubereitung, im Bereich von 1 bis 1.000 mg/kg liegt, vorzugsweise im Bereich von 1 bis 750 mg/kg, besonders bevorzugt im Bereich von 5 bis 500 mg/kg,
ii) Bereitstellen eines oder mehrerer weiterer Bestandteile der herzustellenden Zubereitung, und
iii) Inkontaktbringen oder Mischen der in Schritt ii) bereitgestellten weiteren Bestandteile mit dem/den in Schritt i) bereitgestellten Bestandteil(en), vorzugsweise in einer sensorisch wirksamen Menge.
